(19) Europäisches Patentamt
European Patent Office
Office européen des brevets

(11) Publication number: 0 113 101
A1

(12) EUROPEAN PATENT APPLICATION

(21) Application number: 83112827.7

(22) Date of filing: 20.12.83

(51) Int. Cl.³: C 07 D 487/04
A 61 K 31/40

(30) Priority: 30.12.82 US 454796

(43) Date of publication of application:
11.07.84 Bulletin 84/28

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Christensen, Burton G.
770 Anderson Avenue Apt. 19H
Cliffside Park New Jersey(US)

(72) Inventor: Shih, David H.
2 Colby Court
Manalapan New Jersey(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) 6-(1-Hydroxyethyl)-2-SR8-1-methyl-1-carbadethiapen-2-em-3-carboxylic acid esters.

(57) ~~Disclosed~~ are 6-[1-hydroxyethyl]-2-SR⁸-1-methyl-1-carbadethiapen-2-em-3-carboxylic acid esters (I) which are orally active antibiotics:

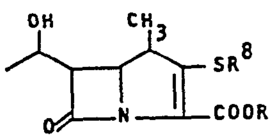

wherein: R is a pharmaceutically acceptable ester moiety consistent with oral delivery; and $R^8$ is substituted or unsubstituted: alkyl, alkenyl, alkynyl, or cyclic alkyl, alkenyl, alkynyl, having 1-6 carbon atoms, aryl such as phenyl or heteroaryl such as pyridyl; wherein the substituent or substituents are selected from: phenyl, pyridyl, cyano, fluoro, chloro, hydroxy, alkylthio such as methylthio, arylthio such as phenylthio, methoxy, phenoxy, alkoxycarbonyl such as methoxycarbonyl, acetoxyl, N-methylcarbamoyl, N-methylcarbamoyloxy and N-acylamino.

Also disclosed are processes for the preparation of such compounds and pharmaceutical compositions comprising such compounds.

EP 0 113 101 A1

## 6-/¯1-HYDROXYETHYL/-2-SR$^8$-1-METHYL-1-CARBADETHIAPEN-2-EM-3-CARBOXYLIC ACID ESTERS

### BACKGROUND OF THE INVENTION

European patent application 81 110 531.1 (Publication No. 0 054 917) is incorporated herein by reference.

This invention relates to 6-[1-hydroxyethyl]-2-SR$^8$-1-methyl-1-carbadethiapen-2-em-3-carboxylic acid esters (I) which are useful as orally active antibiotics:

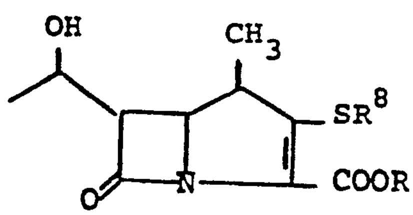

I

wherein: R is a pharmaceutically acceptable ester moiety consistent with oral delivery; and $R^8$ is substituted or unsubstituted: alkyl, alkenyl, alkynyl, or cyclic alkyl, alkenyl, alkynyl, having 1-6 carbon atoms, aryl such as phenyl or heteroaryl such as pyridyl; wherein the substituent or substituents are selected from: phenyl, pyridyl, cyano, fluoro, chloro, hydroxy, alkylthio such as methylthio, arylthio such as phenylthio, methoxy, phenoxy, alkoxycarbonyl such as methoxycarbonyl, acetoxyl, N-methylcarbamoyl, N-methylcarbamoyloxy and N-acylamino. R and $R^8$ are additionally defined below.

This invention also relates to processes for the preparation of such compounds (I); pharmaceutical compositions comprising such compounds; and to methods of treatment comprising administering such compounds and compositions when an antibiotic effect is indicated.

There is a continuing need for new antibiotics. For, unfortunately, there is no static effectiveness of any given antibiotic, because continued wide scale usage selectively gives rise to resistant strains of pathogens. In addition, the known antibiotics suffer from the disadvantage of being effective only against certain types of microorganisms. Accordingly, the search for new antibiotics continues. This search is particularly acute for orally active antibiotics.

Thus, it is an objection of the present invention to provide a novel class of orally active antibiotics which are useful in animal and human therapy. These antibiotics are active against a broad

range of pathogens which representatively include both gram positive bacteria such as S. aureau, Strep. pyogenes, and B. subtilis, and gram negative bacteria such as E. coli, Proteus morganii, Serratia, and Klebsiella. Further objects of this invention are to provide chemical processes for the preparation of such antibiotics; pharmaceutical compositions comprising such antibiotics; and to provide methods of treatment comprising administering such antibiotics and compositions when an antibiotic effect is indicated.

## DETAILED DESCRIPTION OF THE INVENTION

### A. Definition of $R^8$ and R

Relative to the definition of I:

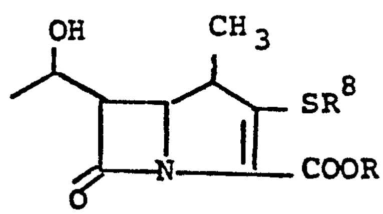

R is an ester moiety consistent with oral delivery which is otherwise pharmaceutically acceptable. The most preferred values for R include:

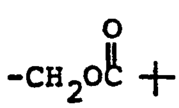

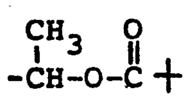

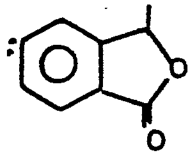

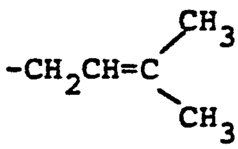

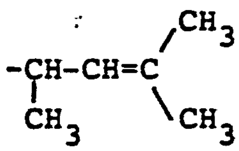

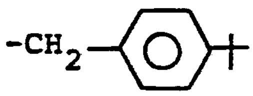

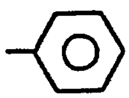

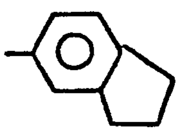

$-CH_2-S-CH_3$

$-CH_2-O\overset{O}{\overset{\|}{C}}OEt$

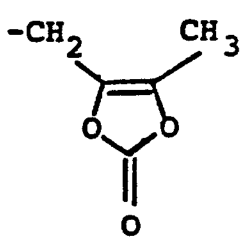

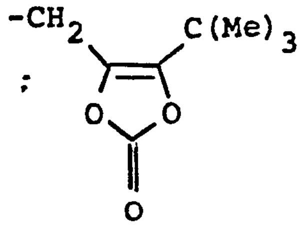

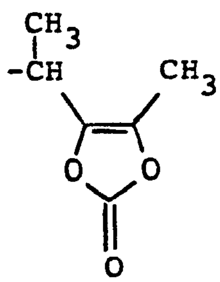

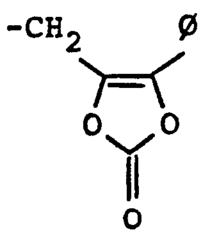

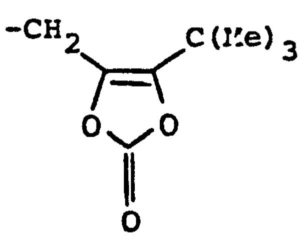

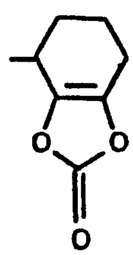

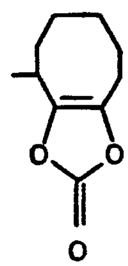

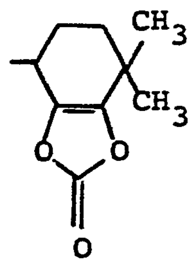

With regard to the definition $R^8$ in Structure I, it has been found that moderately polar to non-polar groups of the above generic design confer the requirements of oral activity. The most preferred values for $R^8$ include:

Methyl (Me)

Ethyl (Et)

$CH(CH_3)_2$

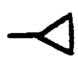

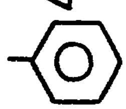

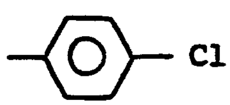

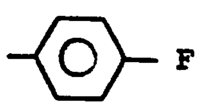

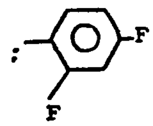

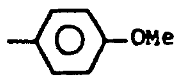

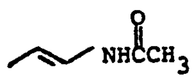

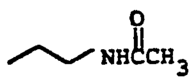

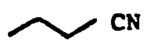

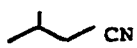

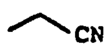

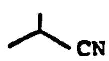

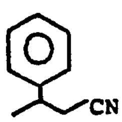

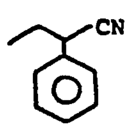

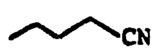

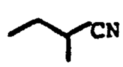

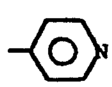

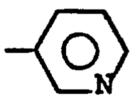

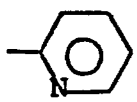

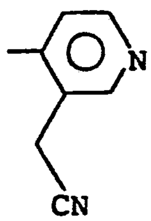

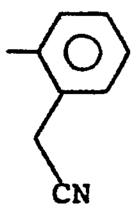

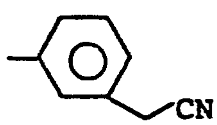

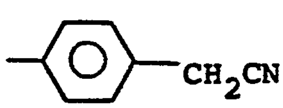

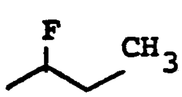

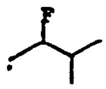

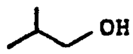

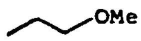

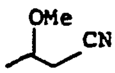

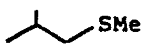

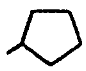

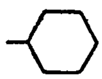

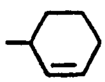

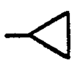

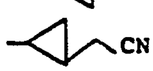

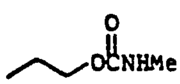

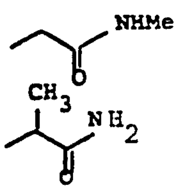

$CH{=}CHCONH_2$
$CH{=}CHCO_2Me$
$CH{=}CHCN$

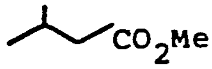

;

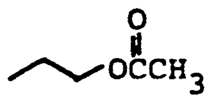

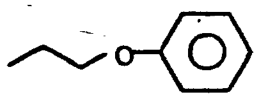

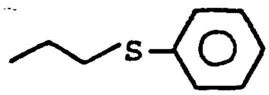

$-CF_3$

$-CH_2C{\equiv}CCH_3$

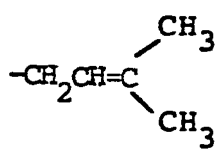

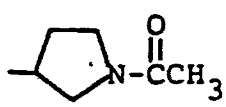

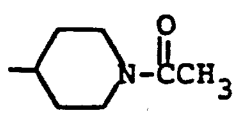

B. Synthesis

The compounds of the present invention are conveniently prepared by any of the following three (3) schemes:

Scheme I

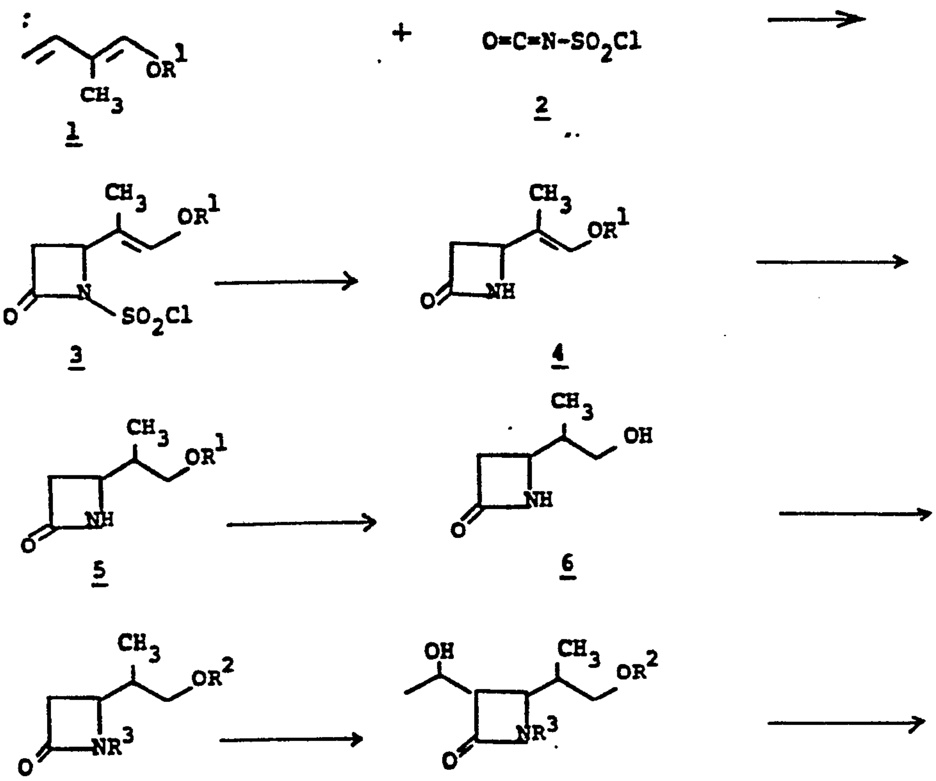

1

2

3

4

5

6

7

8

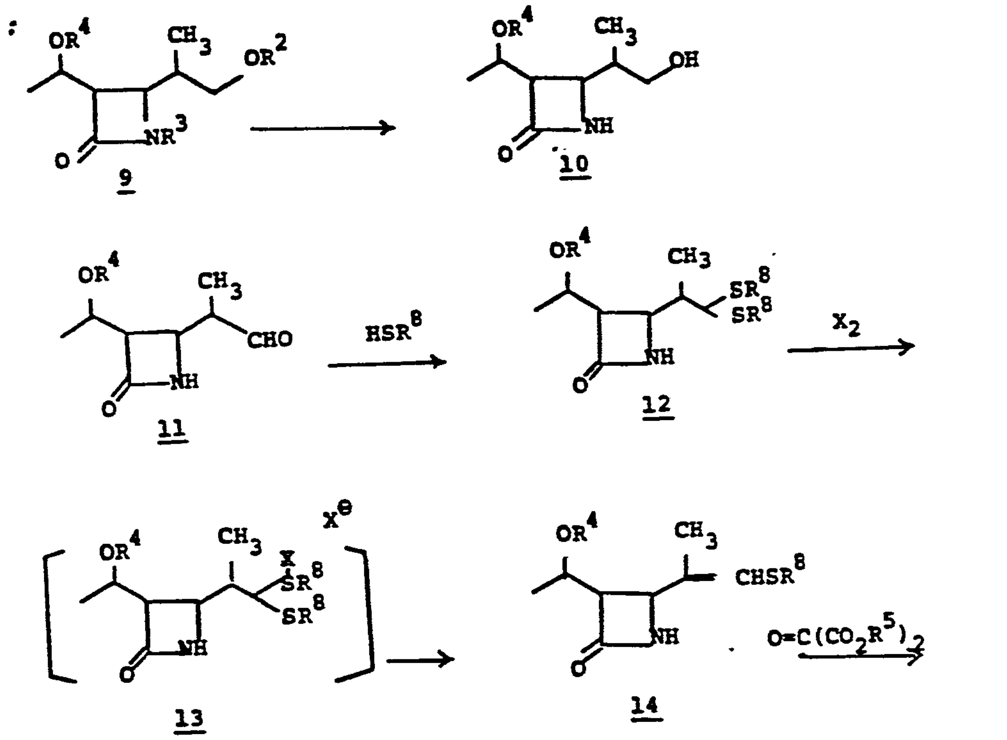


9 10 11 12 13 14

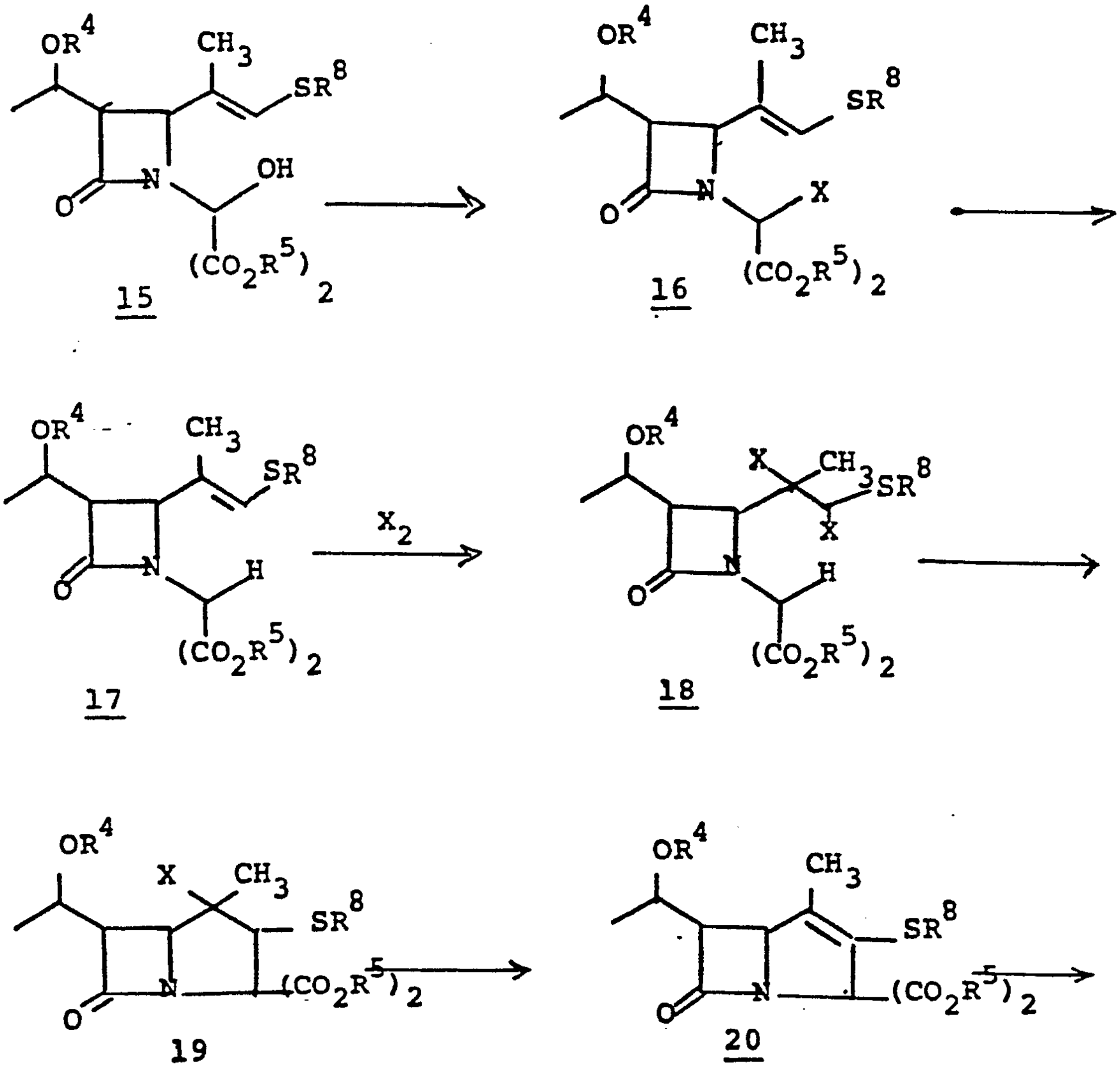


15 16 17 18 19 20

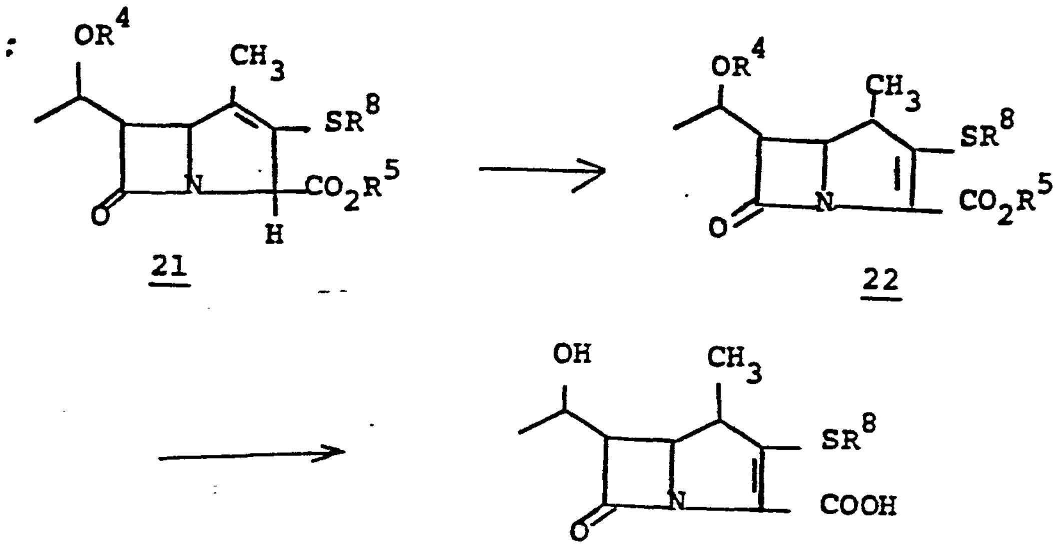

21

22

I

In words relative to the above diagram, the 4-(1,2-substituted-vinyl)azetidine-2-one, 4, starting material is prepared by reacting 1-etoxyl-2-methyl-1,3-butadiene, 1, with chlorosulfonylisocyanate 2. The reaction is conducted without solvent or may be run in solvent such as diethyl ether, ethyl acetate, chloroform, methylene chloride, or the like; at a temperature of from -78°C to 25°C for from a few minutes to 1 hour to provide 3. The radical $R^1$ is an easily removable acyl blocking group such as an alkanoyl or aralkanoyl which bears no functional group or groups which might not interfere with the desired course of reaction (1 + 2 to 3 to 4). Intermediate species 3 is converted to the sulfinamide by reduction which is then hydrolyzed to 4 at pH 6-8. Typically the reaction solution comprising 3 is contacted (5-30 minutes) with an aqueous solution (at 0-25°C) of a reducing agent such as sodium sulfite, thiophenol, or the like at pH 6-8 to provide 4.

The reaction 4 to 5 is a reduction, and is preferably achieved by hydrogenation in a solvent such as ethyl acetate, ether, dioxane, tetrahydrofuran (THF), ethanol, or the like at 0 to 25°C for from 5 minutes to 2 hours under 1 to 10 atmospheres of hydrogen in the presence of a hydrogenation catalyst such as platinum metal or oxide, 10% Pd/C or the like.

The de-blocking reaction 5 to 6 is usually desirable when $R^1$ is acyl to permit the later alkylation, 7 to 8. The preferred de-blocking procedure is by alcoholysis wherein the solvent is a lower alkanol such as methanol, ethanol, or the like in the presence of the corresponding alkali metal alkoxide, such as sodium methoxide. Typically, the reaction is conducted for from 5 minutes to 1 hour at a temperature of from -10° to 25°C.

Blocking groups $R^3$ and $R^2$ are established (6 to 7) to provide a suitably protected species for alkylation (7 to 8 to 9). There is no criticality in the choice of blocking groups, provided only that they do not interfere with the intended alkylation. $R^3$ may be hydrogen, a triorganosilyl group such as trimethylsilyl, t-butyldimethylsilyl or the like; or a cyclic ether such as 2-tetrahydropyranyl; $R^2$ may also be cyclic ether, such as 2-tetrahydropyranyl; alternatively, $R^3$ and $R^2$ may be joined together to form protected species such as 7a:

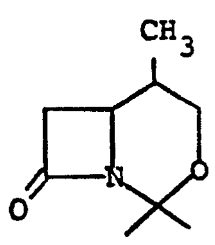

7a

For example, species such as 7a are conveniently prepared by treating 6 with 2,2-dimethoxypropane in the presence of a catalyst such as boron trifluoride etherate, toluene sulphonic acid, or the like in a solvent such as methylene chloride, ether, chloroform, dioxane or the like at a temperature of from -10°C to 35°C for from a few minutes to 1 hour. Compound 7 is treated with a strong base such as lithium diisopropylamide, sodium hydride, phenyl lithium or butyl lithium and the like in a solvent such as tetrahydrofuran (THF), ether, dimethoxyethane and the like at a temperature of from -80°C to 0°C, whereupon the acetaldehyde is added to provide species 8. The hydroxy function of 8 is protected with a protecting groups such as p- or o-nitrobenzyoxycarbonyl by treating 8 with 1.5 eq of p-nitrobenzoxycarbonyl chloride and 2.0 eq of p-dimethylaminopyridine in a solvent such as DMF at room temperature for 30 minutes to 6 hours to give 9.

The de-blocking reaction 9 to 10 is typically conducted by acid hydrolysis such as aqueous acetic acid at a temperature of from 25°C to 75°C for from 5 minutes to 3 hours.

The aldehyde intermediate 11 is prepared by treating 10 with an oxidizing agent such as $CrO_3$.2(pyridine) in $CH_3CN$, 1:1 mixture of dimethylsulfoxide and acetic anhydride, cyclohexylcarbodiimide in DMSO, pyridium chlorochromate in $CH_2Cl_2$, or the like at a temperature of from 0-25°C for from 5 minutes to 1 hour. The resulting species 11 in a solvent such as acetonitrile, methylene chloride, chloroform or the like at a temperature of from -10 to 25°C is treated with an

excess of the reagent $HSR^8$ in the presence of an acid catalyst such as boron trifluoride etherate, toluene sulphonic acid or the like to provide 12. Typically, the reaction requires from 1 to 60 minutes.

The vinyl sulphide 14 is obtained *via* intermediate 13 by treating 12 with a halogen such as chlorine or bromine (X = Cl or Br) in a solvent such as ether, methylene chloride, tetrahydrofuran, glyme or the like at a temperature of from -78° to 30°C for from 1 to 30 minutes followed immediately by treating with an olefin such as cyclohexene, isobutylene, or the like in the presence of base such as triethylamine, DBU, sodium hydride, or the like in a solvent such as DMF, glyme, THF, HMPA. The solution is held at -20° to 25°C for from 1 to 8 hours to yield 14.

The vinyl sulphide species 14 is reacted with a diester of oxomalonic acid (or its monohydrate) to provide 15. There is no criticality as to the identity of the ester moiety, $R^5$, of the oxomalonic acid. $R^5$ may be a conventional, easily removable blocking group or it may be a pharmaceutically acceptable ester moiety. Suitable ester radicals $R^5$ are *p*-nitrobenzyl, benzyl, *o*-nitrobenzyl, t-butyl, 2,2,2-trichloroethyl. The reaction 14 to 15 is typically conducted in an organic solvent such as benzene, toluene, cyclohexane, halo aromatic or the like at a temperature of from about 50°C to reflux for from 0.5 to 6 hours.

The halogenation reaction 15 to 16 is typically conducted in a solvent such as THF, glyme, ether, methylene chloride, chloroform or the like in

the presence of a halogenating agent such as thionyl chloride, phosphorous pentachloride or the like in the presence of base such as pyridine at a temperature of from -20° to 25°C for from 5 minutes to 3 hours. The selective reduction of 15 to 17 via 16 is completed by treating 16 with tributylphosphine, triphenylphosphine, or the like in aqueous DMF or similar aqueous systems involving dioxane, THF, glyme, DMSO, or acetone at a temperature of from about 0-50°C for from 10 minutes to 5 hours.

Species 17 is halogenated by the previous procedure (12 to 13), but omitting the addition of the cyclohexene or other olefin, to provide the dihalo species 18. Species 18 is treated with a base such as triethylamine, sodium hydride or potassium hydride in a solvent such as DMF, acetonitrile, methylene chloride, chloroform, glyme or the like at a temperature of from about -78° to 25°C for 1 to 5 hours to provide 19. Species 19 is converted to 20 on treatment with a strong base such as 1,5-diazabicyclo[5.4.0]undec-5-ene(DBU),1,5-diazabicyclo[3.4.0]non-5-ene(DBN), or the like in a solvent such as DMSO, acetone, chloroform, DMF, THF, glyme or the like or on treatment with AgF in pyridine at a temperature of from 0-40°C for from 1/4 to 24 hours. The reaction 20 to 21 is conducted by treating 20 with an aromatic base such as pyridine, aqueous dimethylsulfoxide, s-collidine or lutidine, in the presence of a displacing agent such as lithium iodide, sodium chloride, lithium bromide, sodium bromide, or the like at a temperature of from about 80-150°C for from 15 minutes to 2 hours. An aqueous work up of the resulting reaction mixture provides

21. Isomerization of the double bond 21 to 22 is accomplished by treating 21 in a solvent such as DMF, DMSO, ethyl ether, THF, glyme, methylene chloride with a strong base such as diisoprypylamine, DBU, DBN, or the like at a temperature of from 0° to about 25°C for from a few minutes to 2 hours or until equilibrium has been established as determined by examination of sample aliquots by ultraviolet absorption or by thin layer chromatography. The final reaction 22 to I (hydrogenolysis of the blocking group) is accomplished by treating 22 in a solvent such as dioxane, ethanol, THF or the like or an aqueous mixture thereof in the presence of a metal catalyst such as Pd/C or platinum under a hydrogen pressure of from 1-4 atmospheres for from 0.5 to 8 hours at a temperature of from about 0-25°C.

It is recognized that $SR^8$ side chains in which the $R^8$ group contains one or more chiral centers can be added as racemic or diastereomeric mixtures to provide mixtures of diastereomeric products which can be separated by conventional chromatography or can be added as resolved, isomerically pure reagents to provide diastereomerically pure products. Since antibacterial activity and other pharmacological properties vary among isomers, it is frequently advantageous to prepare isomerically pure products by the introduction of resolves $-SR^8$ side chains.

Scheme II

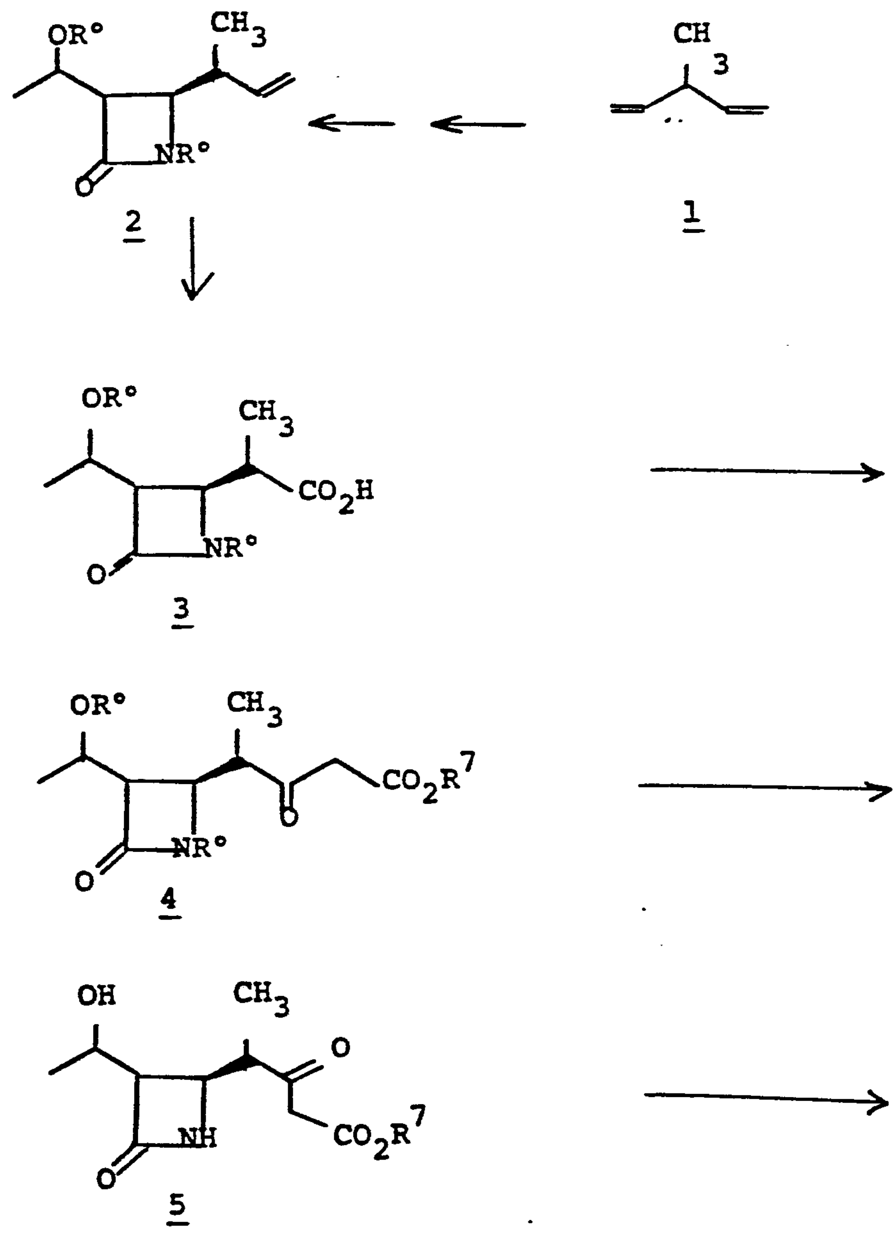

1 2 3 4 5

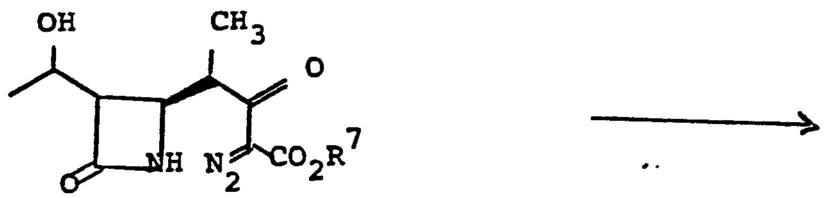

6

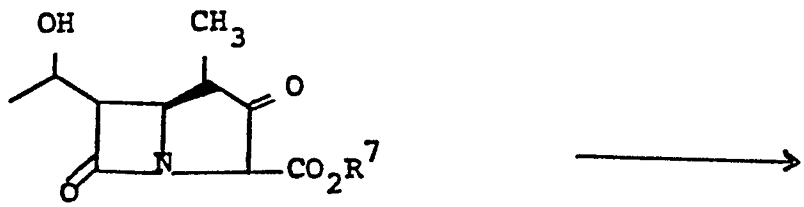

7

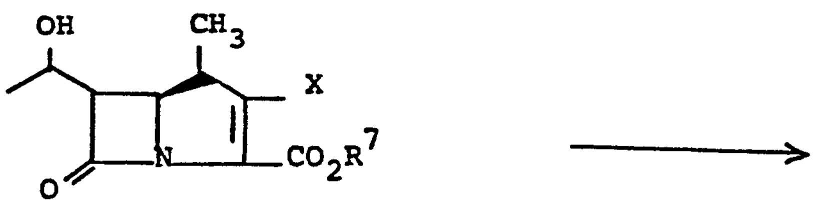

8

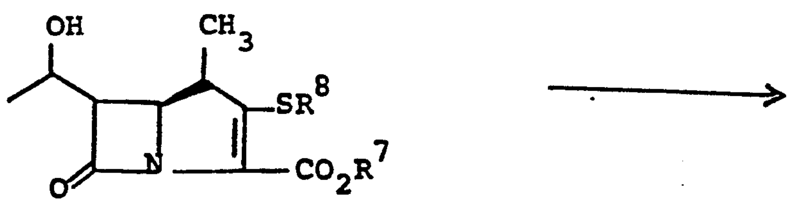

9

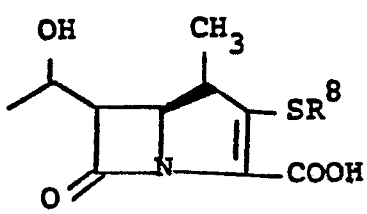

II

In words relative to the diagram, the oxidation 2 to 3 is accomplished by treating 2 in a solvent such as methylenechloride, methanol, chloroform, for the like, with an oxidizing agent such as ozone, or the like, at a temperature of from -100° to 0°C for from 0.1 to 4 hours, followed by treating the crude product with an oxidizing agent such as m-chloroperbenzoic acid, hydrogen peroxide, peracetic acid, or the like, at a temperature of from 0°C to 100°C for from 1 to 100 hours. R° is a readily removable protecting group and is defined below.

The addition 3 to 4 is accomplished by treating 3 with 1,1'-carbonyldiimidazole, or the like, in a solvent such as tetrahydrofuran, dimethoxyethane, acetonitrile or the like, at a temperature of from 0 to 70°C, followed by the addition of 1.1 to 3.0 equivalent of $(R^7O_2CCH_2CO_2)_2Mg$, at a temperature of from 0 to 70°C for from 1 to 48 hours. $R^7$ is a pharmaceutically acceptable ester moiety or a readily removable carboxyl protecting groups such as p-nitrobenzyl, benzyl, or the like.

Removal of protecting group R° (4 to 5) (when R° = t-butyldimethylsilyl) is accomplished by acidic aqueous hydrolysis of 4 in a solvent such as methanol, ethanol, tetrahydrofuran, dioxane, or the like, in the presence of an acid such as hydrochloric, sulfuric, acetic or the like at a temperature of from 0 to 100° for from 2 to 18 hours.

The diazo species 6 is prepared from 5 by treating 5 in a solvent such as $CH_3CN$, $CH_2Cl_2$, THF, or the like with an azide such as p-carboxy-

benzenesulfonylazide, toluenesulfonylazide, methanesulfonylazide, or the like in the presence of a base such as triethylamine, pyridine, diisopropylethylamine or the like for from 1 to 50 hours at 0-50°C.

Cyclization (6 to 7) is accomplished by treating 6 in a solvent such as benzene, toluene, THF, cyclohexane, ethylacetate or the like at a temperature of from 25 to 110°C for from 1-5 hours in the presence of a catalyst such as *bis* (acetylacetonato)Cu(II) [$Cu(acac)_2$], $CuSO_4$, Cu powder, $Rh(OAc)_2$ or $Pd(OAc)_2$. Alternatively, the cyclization may be accomplished by irradiating 6 through a pyrex filter (a wave length greater than 300nm) in a solvent such as benzene, $CCl_4$, diethylether, or the like, at a temperature of from 0-25°C for from 0.5 to 2 hours. ["OAc" = acetate.]

Establishment of leaving group X (7 to 8) is accomplished by acylating the keto ester 7 with an acylating agent RX such as p-toluenesulfonic acid anhydride, p-nitrophenylsulfonic acid anhydride, 2,4,6-triisopropylphenylsulfonic acid anhydride, methanesulfonic acid anhydride, trifluoromethanesulfonic acid anhydride, toluenesulfonyl chloride, p-bromophenylsulfonyl chloride, diphenylchlorophosphate, or the like; wherein X is the corresponding leaving group such as toluenesulfonyloxy, p-nitrophenylsulfonyloxy, methanesulfonyloxy, p-bromophenylsulfonyloxy and other leaving groups which are established by conventional procedures and which are well known in the art. Typically, the above acylation to establish leaving groups X is conducted in a solvent such as methylene chloride, acetonitrile or dimethylformamide, in the presence of a base such as diisopropylethylamine,

triethylamine, 4-dimethylaminopyridine or the like at a temperature of from -20 to 40°C for from 0.5 to 5 hours. The leaving group X of intermediate 8 can also be halogen. The halogen leaving group is established by treating 7 with a halogenating agent such as $O_3PCl_2$, $O_3PBr_2$, $(ØO)_3PBr_2$, oxalyl chloride, $PBr_3$ or the like in a solvent such as $CH_2Cl_2$, $CH_3CN$, THF, or the like in the presence of a base such as diisopropylethylamine, triethylamine, or 4-dimethylaminopyridine or the like. [Ø = phenyl.]

The reaction 8 to 9 is accomplished by treating 8 in a solvent such as dioxane, dimethylformamide, dimethylsulfoxide, acetonitrile, hexamethylphosphoramide, or the like in the presence of an approximately equivalent to excess of the mercaptan reagent $HSR^8$ or its salt, $R^8S^-M^+$ (M=Na, K or the like) wherein $R^8$ as defined above. A representative mercaptan reagent is $HSCH_2CH_2NHR^{8'}$ wherein $R^{8'}$ is hydrogen or a readily removable N-protecting group such as p-nitrobenzyloxycarbonyl, o-nitrobenzyloxycarbonyl, or the like in the presence of a base such as sodium hydrogen carbonate, potassium carbonate, triethylamine, diisopropylethylamine, or the like at a temperature of from -40 to 25°C for from 1 to 72 hours. The mercaptan reagent, $HSCH_2CH_2NHR^{8'}$, is typically prepared by treating aminoethylmercaptan in the presence of the desired acid chloride in the presence of a base such as sodium bicarbonate, sodium hydroxide, or the like in a solvent such as aqueous diethylether, aqueous dioxane, aqueous acetone, or the like at a temperature of from 0 to 25°C for from 0.5 to 4 hours.

The final deblocking step 9 to II is accomplished by conventional procedures such as hydrolysis or hydrogenolysis. Typically 9 in a solvent such as dioxane-water-ethanol, tetrahydrofuran-aqueous dipotassium hydrogen phosphate-isopropanol or the like is treated under a hydrogen pressure of from 1 to 4 atmospheres in the presence of a hydrogenation catalyst such as palladium on charcoal, palladium hydroxide, or the like at a temperature of from 0 to 50°C for from 0.5 to 4 hours to provide II.

Preparation of Starting Material 1 and 2

With respect to starting reagent 1, its preparation is generally described in *J. Amer. Chem. Soc.*, 74, 661 (1952) by E. B. Reid and T. E. Gompf, *J. Org. Chem.*, 23, 1063 (1958) by R. Ciola and K. L. Burwell, Jr., and Belgium Patent 632, 193 (1963) by R. Polster and E. Scharf. The following scheme summarizes the preparation of 1.

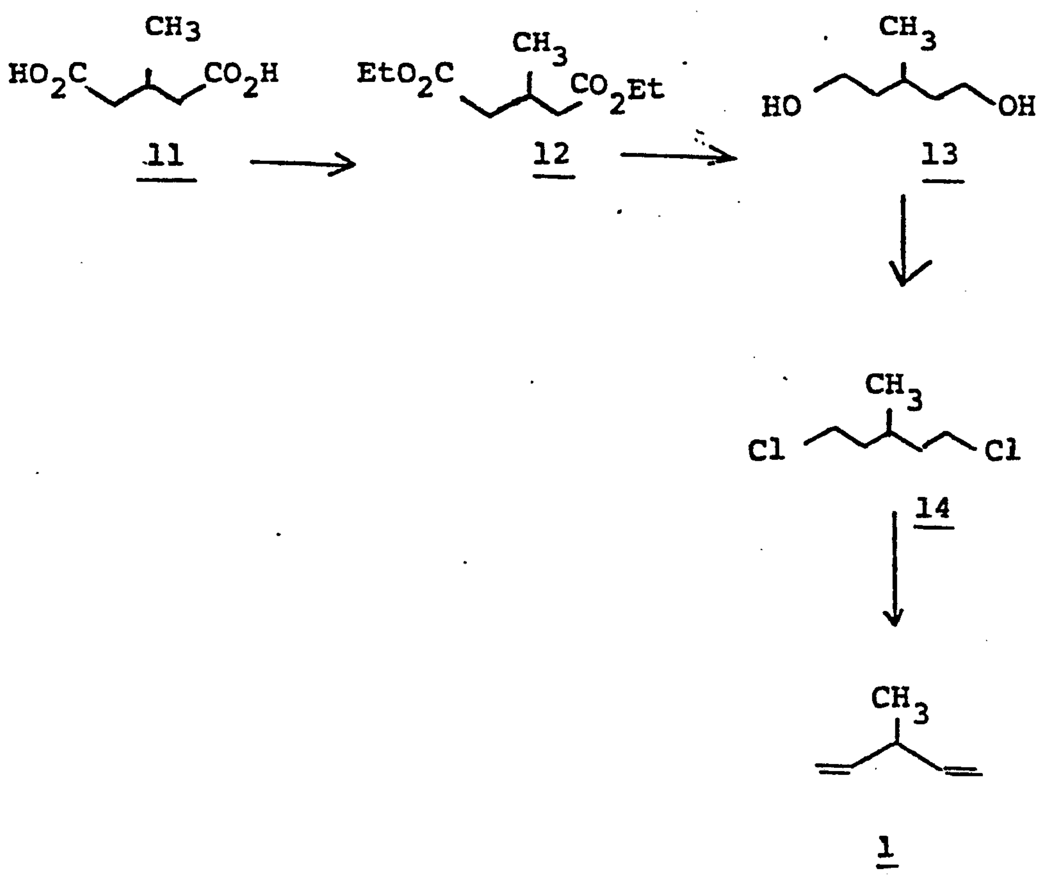

11 12 13 14 1

In words relative to the above scheme, the diester 12 is prepared by treating the diacid 11 with thionyl chloride at reflux for two hours followed by reacting with ethanol at 80°C for 4 hours. Reduction of the diester 12 with lithium alumium hydride in ether at reflux for 4 hours followed by hydrolysis with 10% NaOH gives diol 13 which on further reaction with thionyl chloride gives dichloride 14. Treatment of the dichloride 14 with base such as 2-methylquinoline, DBU or sodium hydroxide in polyethylene glycol gives the expected 3-substituted 1,4-pentadiene 1.

Preparation of 2 is summarized in the following scheme:

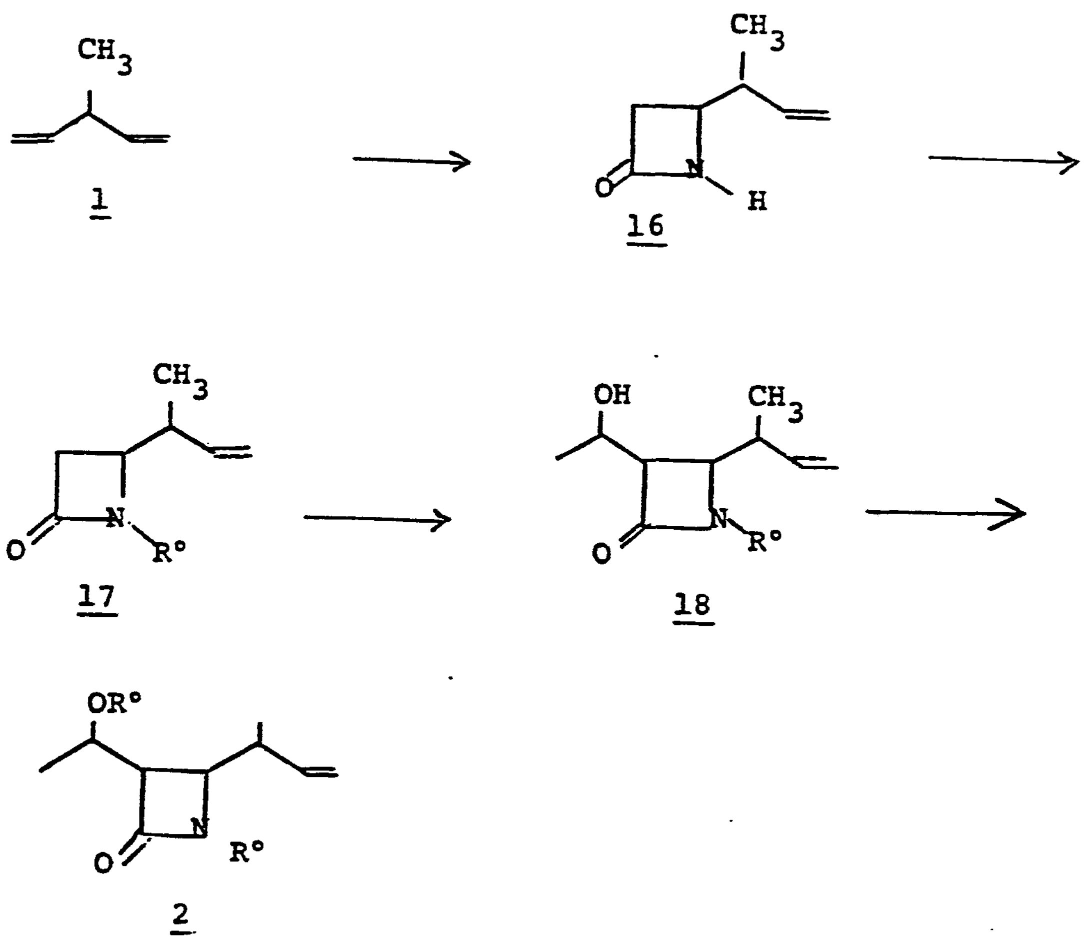

1

16

17

18

2

In words relative to above scheme, the methyl azetidinone 16 is prepared by reacting 3-methyl-1,4-pentadiene 1 with chlorosulfonylisocyanate at 25°C to 60°C in a pressure bottle for 3-12 days, then the resulting mixture is hydrolyzed with aqueous sodium sulfite solution between pH 6.5-7.5 at 0°C to 25°C for from 5 minutes to 60 minutes.

Azetidinone 16 is transformed (16 to 17) to establish the protecting group R° which may be a triorganosilyl group, such as t-butyldimethylsilyl, t-butyldiphenylsily, triphenylsilyl, isopropyldimethylsilyl, for example, or may be 3,4-dimethoxybenzyl, for example. Silyl protection is preferred, and typically R° is established by treating 16 in a solvent such as dimethylformamide, acetonitrile, hexamethylphosphoramide, tetrahydrofuran and the like with a silylating agent such as t-butyldimethylchlorosilane, t-butyldiphenylchlorosilane, triphenylchlorosilane, and the like at a temperature of from -20° to 25°C for from 0.5 to 24 hours in the presence of a base such as triethylamine, diisopropylethylamine, or the like.

Aldol condensation of 17 with acetaldehyde provides 18. Typically, 17 is treated with a strong base such as lithium diisopropylamide, sodium hydride, phenyl lithium or butyl lithium and the like in a solvent such as tetrahydrofuran (THF), ether, dimethoxyethane and the like at a temperature of from -80°C to 0°C, whereupon acetaldehyde is added to provide species 18. To establish the hydroxy protecting group R° (18 to 2) Azetidinone 18 is treated with t-butyldimethylchlorosilane in dimethylformamide in the presence a base such as imidazole, triethylamine or the like at a temperature of from 0° to 60°C for from 1 to 16 hours.

The chiral azetidinone carboxylic acid 3 of Scheme II can be conveniently prepared by methylation of azetidinone carboxylic ester 19 with iodomethane at low temperature -78° to -30°C in the presence of 2 eq of strong base such as lithium diisopropylamide or the like in a solvent such as THF, DMF, HMPA or the like for from 10 minutes to 1 hour. The isomer separation of 20 can be achieved by conventional chromatography or HPLC. Saponification of 20 with 1 eq 10% NaOH in methanol/water at room temperature for 1 hour to 24 hours provides desirable species 3.

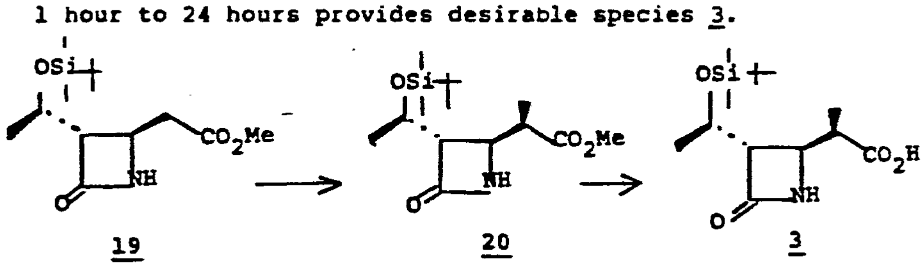

19 20 3

In the foregoing description of the invention, suitable reagents $HSR^8$, its salt, $R^8S^-M^+$ (M=Na, K or the like) (11 to 12 of Scheme I) and (8 to 9 of Scheme II) are representatively illustrated by the following list:

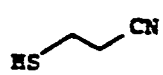

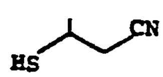

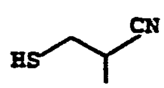

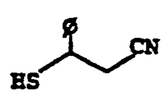

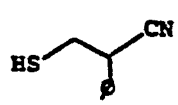

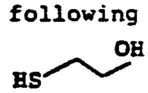

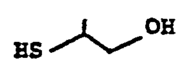

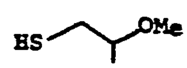

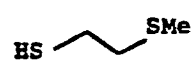

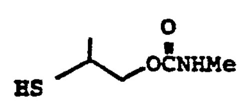

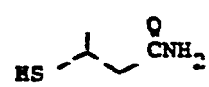

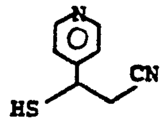

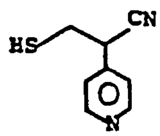

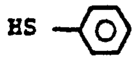

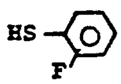

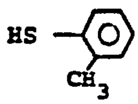

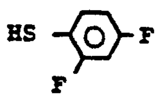

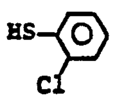

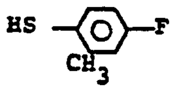

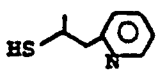

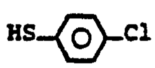

$Na^{\oplus}S^{\ominus}CH=CHCONH_2$

$Na^{\oplus}S^{\ominus}CH=CHCO_2Me$

$Na^{\oplus}S^{\ominus}CH=CHCN$

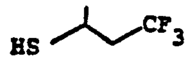

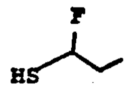

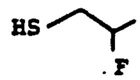

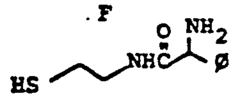

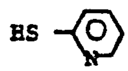

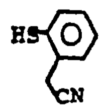

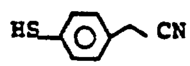

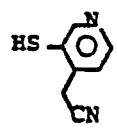

$HSCF_3$

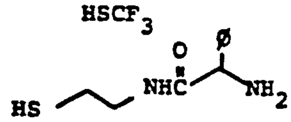

Ø = phenyl

Scheme III

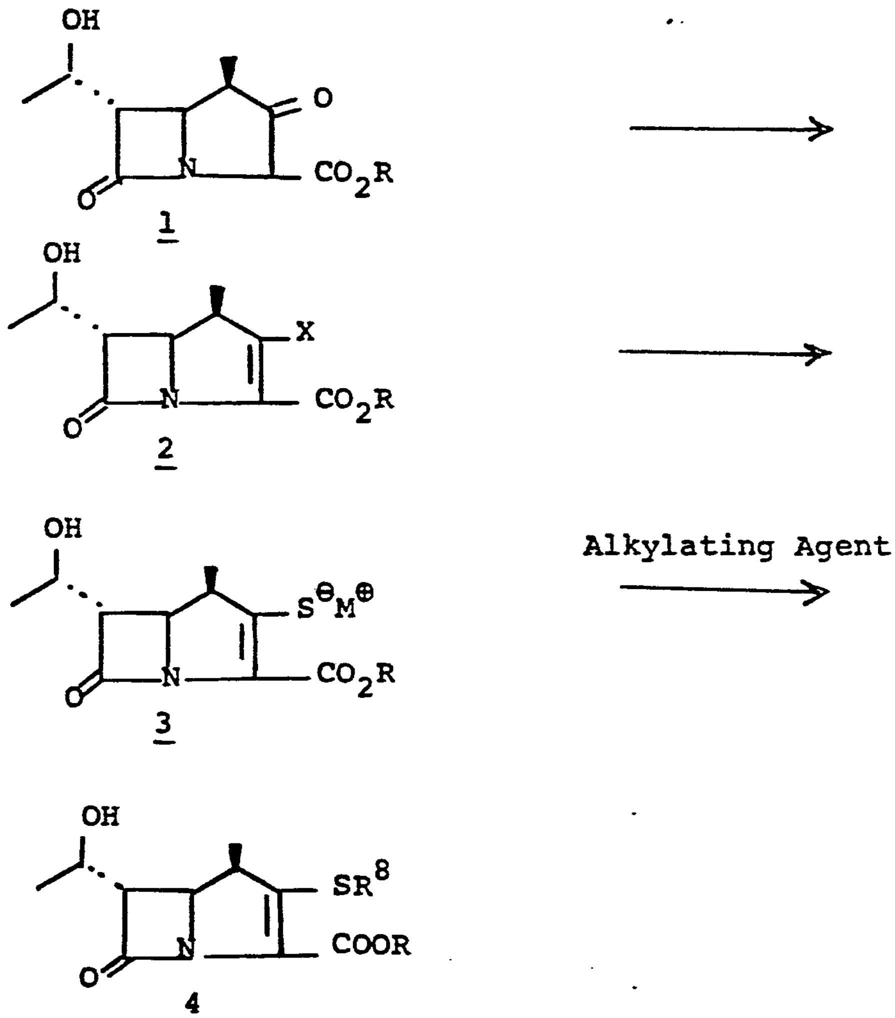


1

2

3

4

In words relative to Scheme III, the leaving group X of 2 is established by the same method described in the preparation of species 8 in Scheme II. The typical leaving group X of 2 used for converting 2 to 3 is a phosphate, such as diphenylphosphate or the like. Thus, the vinyl phosphate 2 in a solvent such as DMF is treated with 1-2 eq of sodium hydrogen sulfide at a temperature of from -50° to 0°C for from 10 minutes to 3 hours in the presence of 1 to 1.5 eq of base such as diisopropylethylamine to give the thio enolate 3. Treatment of 3 with an alkylating agent, $R^8X'$ ($R^8$ is as described above and X' is chloro, bromo, iodo, tosylate, mesylate or the like) or conjugated olefin or acetylene reagents, such as:

$HC\equiv C\overset{\overset{O}{\|}}{C}NHz$, $HC\equiv C\overset{\overset{O}{\|}}{C}OMe$, $HC\equiv CCN$, $H_2C=CH\overset{\overset{O}{\|}}{C}NH_2$

$H_2C=CH\overset{\overset{O}{\|}}{C}OMe$, $H_2C=CHCN$, or the like, at a temperature of from -78° to 0°C for from 10 minutes to 2 days provides the product I.

Representative "Alkylating Agents" for Scheme III:

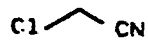

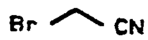

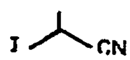

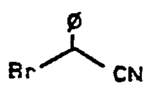

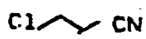

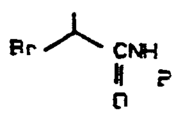

$I-CH_2CH_3$

$ICH_3$

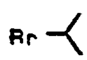

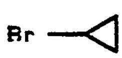

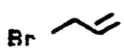

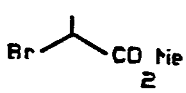

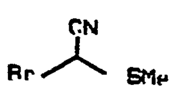

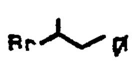

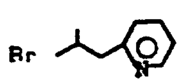

$XCH_3$

$XCH_2CH_3$

$XCH(CH_3)_2$

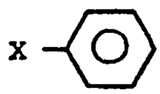

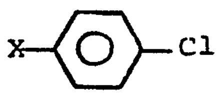

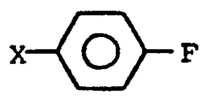

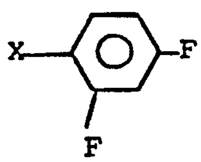

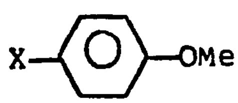

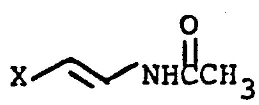

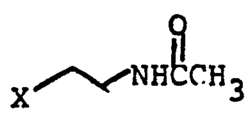

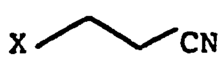

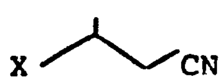

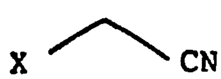

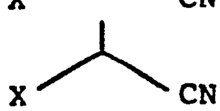

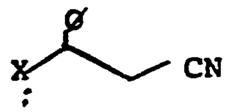

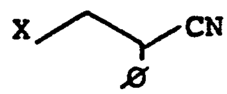

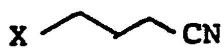

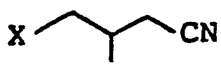

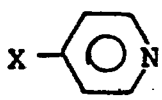

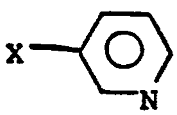

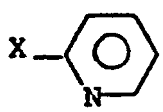

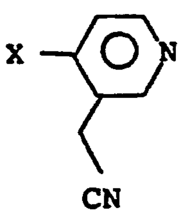

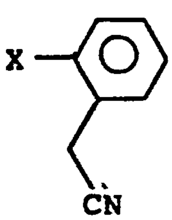

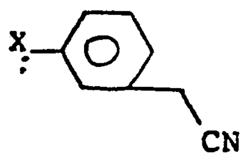

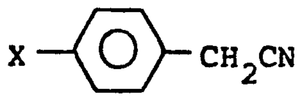

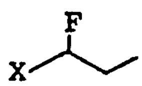

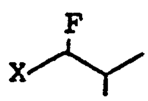

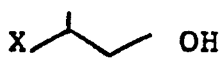

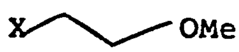

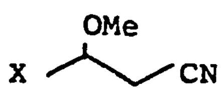

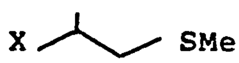

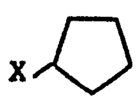

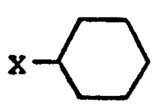

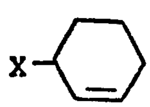

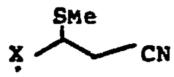

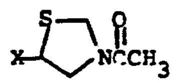

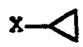

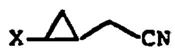

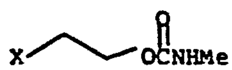

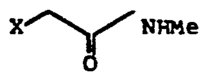

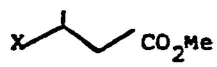

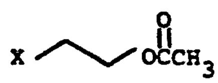

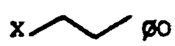

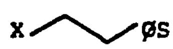

$X-CF_3$

$X-CH_2C{\equiv}CCH_3$

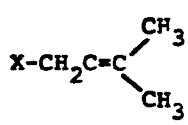

X is a leaving group, such as bromo, chloro, iodo, tosylate, mesylate or the like; Ø is phenyl.

$HC\equiv C-CONH_2$

$HC\equiv C-CO_2Me$

$HC=C-CN$

$H_2C=CHCONH_2$

$H_2C=CHCO_2Me$

$H_2C=CHCN$

$H-\overset{CH_3}{\overset{|}{C}}=CHCN$

$CH_3C\equiv C-CONH_2$

Esterification and Identification of R

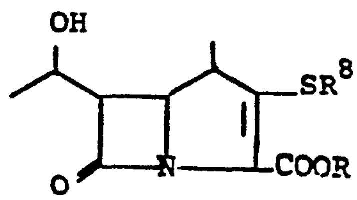

Esterification of carbapenem carboxylic acid is accomplished by treating the alkali salt of the desirable carbapenem 1 with a alkylating agent RX' (R is a bio-labile ester as defined below and X' is a leaving group such as chloride,

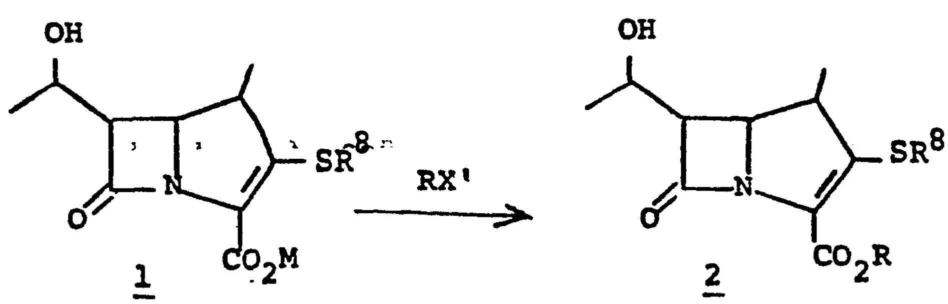


1 2

bromide, iodide, tosylate, mesylate, phosphate or the like) in a solvent such as DMF, DMSO, HMPA or the mixed solvents such as DMF/HMPA and the like at a temperature of from 0°C to 60°C for from 30 minutes to 6 hours.

Examples of the suitable $RX^1$ reagents for the preparation of orally active carbapenem ester 2 are:

1-chloromethylethylcarbonate
chloromethyl methyl ether
chloromethylpivalate (pivaloyloxymethyl chloride)
bromomethylpivalate
1-chloroethylpivalate
1-bromoethylpivalate
1-chloroethylethylcarbonate
chlorophthalide
bromophthalide
4-chloromethyl-1,3-dioxolen-2-one
4-bromomethyl-1,3-dioxolen-2-one
4-chloromethyl-5-methyl-1,3-dioxolen-2-one
4-chloromethyl-5-phenyl-1,3-dioxolen-2-one
4-chloromethyl-5-(t-butyl)-1,3-dioxolen-2-one
4-bromomethyl-5-methyl-1,3-dioxolen-2-one
4-iodomethyl-5-methyl-1,3-dioxolen-2-one
4-(1-bromoethyl)-5-methyl-1,3-dioxolen-2-one
3-chloro-1,2-carbonyldioxycyclohexene
3-bromo-1,2-carbonyldioxycyclohexene
3-bromo-6,6-dimethyl-1,2-carbonyldioxycyclohexene
3-chloro-1,2-carbonyldioxycyclooctene

3-bromo-1,2-carbonyldioxycyclooctene
4-bromo-2-methyl-2-butene
4-bromo-2-methyl-2-pentene
p-t-butylbenzylchloride
chloromethylmethylsulfide
chloromethylethylsulfide
iodobenzene
5-bromoindane

Relative to the generic description of the compounds of the present invention, I, the following drawing depicts the most preferred configuration: 5R, 6S, 8R, 1ß-methyl.

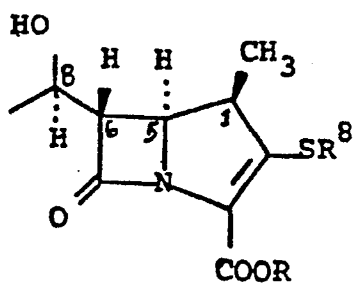

The compounds of the present invention (I) are valuable antibiotics active against various Gram-positive and Gram-negative bacteria and accordingly find utility in human and veterinary medicine. Representative pathogens which are sensitive to antibiotics I include: Staphylococcus aureus, Escherichia coli, Klebsiella pneumoniae, Bacillus subtilis, Salmonella typhosa, and Bacterium proteus. The antibacterials of the invention are limited to utility as medicaments.

The products of this invention may be used in any of a variety of pharmaceutical preparations. They may be employed in capsule, powder form, in liquid solution, or in suspension. They may be administered orally.

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch, acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose.

The compositions may also be prepared in suitable forms for absorption through the mucous membranes of the nose and throat or bronchial tissues and may conveniently take the form of liquid sprays or inhalants, lozenges, or throat paints.

The dosage to be administered depends to a large extent upon the condition and size of the subject being treated as well as the route and frequency of administration. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg.

In the foregoing word description, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents.

The following examples recite a precise scheme of synthesis. It is to be understood that the purpose of this recitation is to further illustrate the invention and not to impose any limitation. Temperature is in °C.

EXAMPLE 1

Preparation of Pivaloyloxymethyl 6-(1-hydroxyethyl)-1-methyl-2-ethylthio-1-carbapen-2-em-3-carboxylate

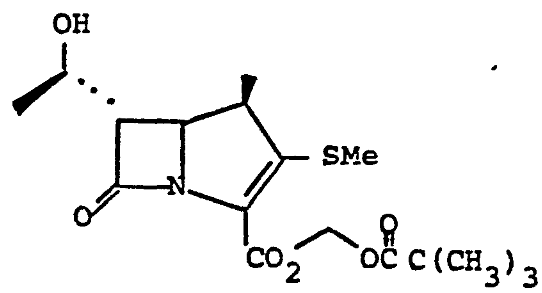

Step A:

Preparation of 1'

$CH_3CHO + CH_3CH_2CHO$

NaOH

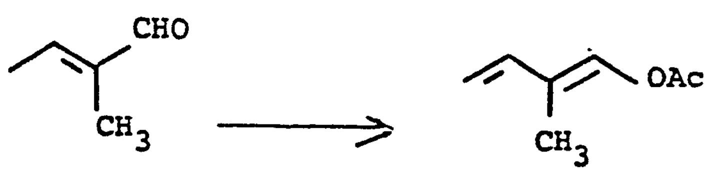

1'

The α,β-unsaturated aldehydes (C) are prepared by modified procedures reported by M.B. Green and W. J. Hickinbottom in J. Chem. Soc. 3262 (1957); and W. J. Bailey and R. Barclay Jr., J. Org. Chem., 21, 328 (1956).

Acetaldehyde (1 eq.) and propionaldehyde (1 eq.) are placed in a three-necked round-bottom flask

which is equipped with a mechanical stirrer, a dry-ice condenser, and a pressure equalized dropping-funnel. To the solution is added dropwise 1 eq. of 1N NaOH through the dropping funnel with constant stirring. After completion of the mixing, the mixture is stirred for 10 minutes, then poured into a beaker containing crushed ice. Extraction of the mixture with ether gives crude product. The desired product (C) is obtained by fractional distillation through a Widmer column.

Isopropenyl acetate (2 eq), cupric acetate (0.002 eq) p-toluenesulfonic acid (0.008 eq.) and the α,β-unsaturated aldehyde C (1 eq.) are placed in a three-necked round-bottom flask equipped with a thermometer, a nitrogen inlet tube and a Widmer column which is attached with a distillation head. The mixture is heated at 93-110°C until quantitative acetone is collected. The mixture is then allowed to cool to r.t. and filtered from solids. The dark brown filtrate is mixed with triethanolamine in water at 0°C. The two layer mixture is distilled quickly under reduced pressure.

The organic layer of the distillate is separated. The aqueous layer is extracted with 200 ml ether. The combined organic layer is washed with 10% $K_2CO_3$, dried over $Na_2SO_4$, and evaporated *in vacuo*. The residue so obtained is mixed with 2.0 g N-phenyl-β-naphthamine and distilled under reduced pressure to give the desired methyl 1-acetoxy-1,3-butadiene (1').

Step B

Preparation of 2' and 3'

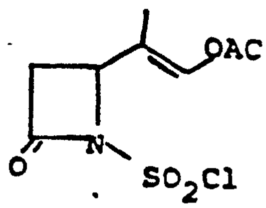

2'

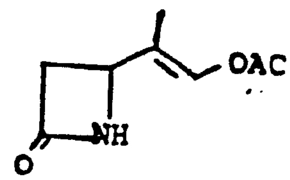

3'

Chlorosulfonylisocyanate (CSI) (6.5 ml) is placed in a three-necked, 100-ml flask equipped with a thermometer, a magnetic stirring bar, a nitrogen inlet tube and a 25-ml pressure-equalized dropping funnel. The CSI is chilled to -50°C and mixed with 12.5 ml ether through the dropping funnel. The etheral solution of CSI is allowed to warm up to -25°C; to the solution is added dropwise 1-acetoxyl-2-methyl-1,3-butadiene (1') (5.9 ml in 12.5 ml ether) in 30 minutes. The mixture is then stirred for 20 minutes at -20 ± 3°C. The white precipitate formed initially is redissolved at the end of the reaction.

In a 500-ml round bottom flask, a solution of 10 g sodium sulfite and 25 g potassium hydrogen phosphate in 100 ml water is prepared and is cooled in an ice bath. Ether (100 ml) and crushed ice (200 g) are added and the mixture is vigorously stirred in an ice bath. At the end of 20 minutes reaction time, the reaction mixture which contains 2' is transferred into the dropping funnel and added dropwise to the hydrolysis mixture in 5 minutes. The hydrolysis is allowed to continue for an additional 30 minutes at

3°C. The organic layer is separated and the aqueous is extracted with 50 ml ether. The organic layers are combined, dried over $Na_2SO_4$ and evaporated to give crystalline product 3' (2.3 g), m.p. 77-78.5°; m.s. 169 ($M^+$); IR 1760 $cm^{-1}$ (β-lactam); NMR (300 MHz, $CDCl_3$): 1.70 (d), 2.16 (s), 2.84 (qq), 3.18 (qq), 4.20 (m), 5.82 (broad, and 6.26 (s) ppm.

Step C

Preparation of 4':

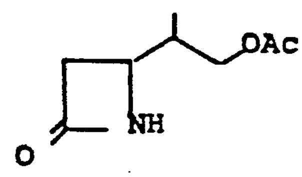

4-(1-methyl-2-acetoxyvinyl)azetidine-2-one (3') (6.5 g) is hydrogenated on a Parr shaker at r.t. under 40 psi hydrogen in the presence of 10% Pd/C (0.6 g) in 200 ml ethylacetate for 2 hours. The mixture is filtered from the catalyst and the filtrate is evaporated in vacuo to give the crude product. Purification of the crude product by high pressure liquid chromatograph (HPLC) (silical gel column, 30% ethylacetate/$CH_2Cl_2$ solvent system) affords a white crystalline product 4' (6.04 g) after evaporation of solvent. The product shows following physical characteristics: ms 171 ($M^+$); IR (Neat) 1754 $cm^{-1}$; NMR (60 MHz, $CDCl_3$): 0.06 (d), 1.01

(d), 2.06 (d, OAc), 2.75-3.80 (m), 3.99 (d) and 6.80 (broad) ppm.

Step D

Preparation of 5':

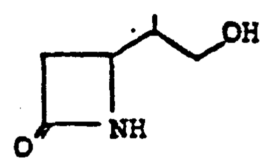

5'

Under $N_2$ at 0° a solution of 4-(1-methyl-2-acetoxyethyl)-2-azetidinone 4' (1.2 g) in 10 ml methanol is treated with sodium methoxide (57 mg). After stirring for 1 hour, the solution is neutralized with glacial acetic acid (65 mg). Removal of methanol *in vacuo* gives crude 4-(1-methyl-2-hydroxyethyl)-2-azetidinone (5') as an oil. The product is purified by chromatography on silica gel eluting with ethyl acetate to give 0.78 g of 5':
IR (neat): 1740 $cm^{-1}$;
NMR ($CDCl_3$): 0.77 (d), 0.96 (d), 1.90 (m), 2.60-3.30 (m), 3.60 (m), 4.19 (s), and 7.23 (s). The product crystallizes as colorless solids in the refrigerator.

Step E

Preparation of 6'

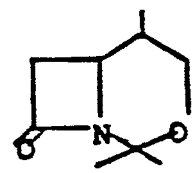

6'

A solution of 4-(1-methyl-2-hydroxyethyl)-2-azetidinone (0.5 g) and 2,2-dimethoxypropane (0.48 g) in 10 ml anhydrous methylene chloride is treated with boron trifluoride (55 mg) at room temperature for 90 minutes. The mixture is washed with 5 ml saturated $NaHCO_3$. The organic layer is separated, dried over $Na_2SO_4$ and allowed to evaporate *in vacuo* to give crude isomeric mixture of 6' (0.48 g) as an oil.

Separation of isomers 6'α and 6'β is accomplished by high pressure liquid chromatograph (HPLC) (silica gel) eluting with 40% ethylacetate/hexanes. After evaporation of the solvents affords 150 mg of 6'β as an oil and 200 mg of 6'α as a white solid.
NMR (300 MHz, $CDCl_3$) of 6'α: 0.81 (d), 1.31 (s), 1.68 (s), 1.62 (m), 2.52 (q), 3.05 (m), 3.42 (t), and 3.66 ppm (q), NMR (300 MHz, $CDCl_3$) of 6'β: 1.10 (d), 1.38 (s), 1.67 (s), 1.90 (m), 2.80 (q), 2.86 (q), 3.62 (q), 3.78 (m) and 3.98 (q) ppm.

Step Fa

Preparation of 7'a

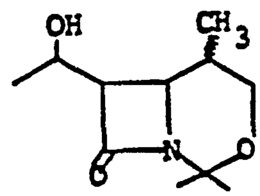

7'α

At -78°C, diisopropylamine (2.2 g) in 20 ml of anhydrous tetrahydrofuran is treated with n-butyllithium (1.6 M in n-hexane, 14 ml) for 5 min. To the solution is add 8-oxo-5α, 2,2-trimethyl-1-azabicyclo[4.2.0]octane (6'a) (3.4 g) and the mixture is stirred for 10 minutes. The resulting lithium enolate is treated with acetaldehyde (1.68 ml). The mixture is stirred for 1 minute then is quenched with 24 ml saturated ammonium chloride at -78°C, then allowed to warm to room temperature (25°C). The mixture is extracted with ethylacetate (2 x 100 ml). The organic layer is separated, dried over $Na_2SO_4$ and allowed to evaporate *in vacuo* to give 4.5 g of the crude product 7'α.

The crude isomeric mixture of 7'α is purified and separated by HPLC (silca gel) eluting with 50% ethylacetate/methylene chloride to give 3.5 g of trans-7'α and 0.5 g of a cis-7'α. Both isomers are crystalline solids.

Step Fb

Preparation of 7'β

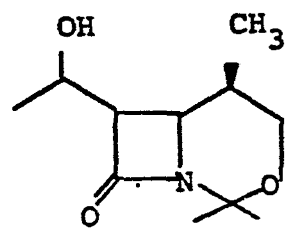

7'β

Following the procedure of Step Fa, except replacing the starting material 6'α with 6'β isomer, the products, trans-7'β (4.0 g) and cis-7'β (0.1 g), are obtained.

Step Fc

Preparation of 7"β

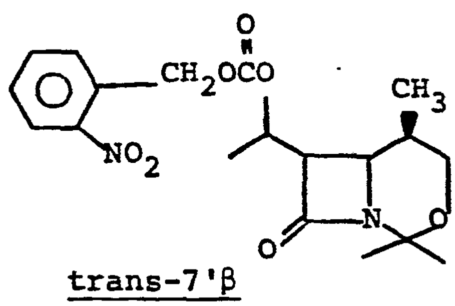

trans-7'β

Under anhydrous conditions at 0°C a solution of R trans-7'β (2.90 g) in 60 ml methylene chloride is treated 4-dimethylaminopyridine (3.32 g) and p-nitrobenzylchloroformate (5.88 g). The mixture is allowed to warm to room temperature and stirred for 1 hour. The resulting mixture is washed with

0.1N Hcl, water, brine and water. The organic layer is separated, dried over $Na_2SO_4$ and allowed to evaporate *in vacuo* to give crude products. The crude products dissolved in 20 ml ether and chilled at -5°C give the o-nitrobenzyl alcohol (0.5 g) which is separated by filtration. The isomeric mixture trans-7"β is purified and separated by HPLC (silica gel) eluting with 40% ethylacetate/cyclohexane to give 1.2 g of S-trans-7"β and 1.0 g of R-trans-7"β.

The spectra data of R-trans-7"β: NMR (300 MHz, $CDCl_3$): 1.12 (d), 1.40 (s), 1.46 (d), 1.73 (s), 1.95 (m), 3.20 (q), 3.60 (q), 3.74 (q), 3.95 (q), 5.07 (m), 5.58 (q), 7.56 (t), 7.70 (m) and 8.19 (d) ppm.

The spectra data of S-trans-7"β: NMR (300 MHZ, $CDCl_3$): 1.10 (d), 1.40 (s), 1.43 (d), 1.72 (s), 1.94 (m), 3.34 (q), 3.61 (q), 3.67 (q), 3.96 (q), 5.13 (m), 5.64 (d), 7.53 (m), 7.68 (m), and 8.17 (d) ppm.

Step Fd

Preparation of 7"α

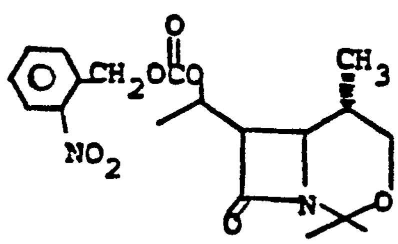

7"α

Following the procedure of Step Fc; except replacing the starting material trans-7'β with trans-7'α isomer, the products R-trans-7"α and S-trans-7"α are obtained.

The following chart summarizes the foregoing separation:

TABLE II

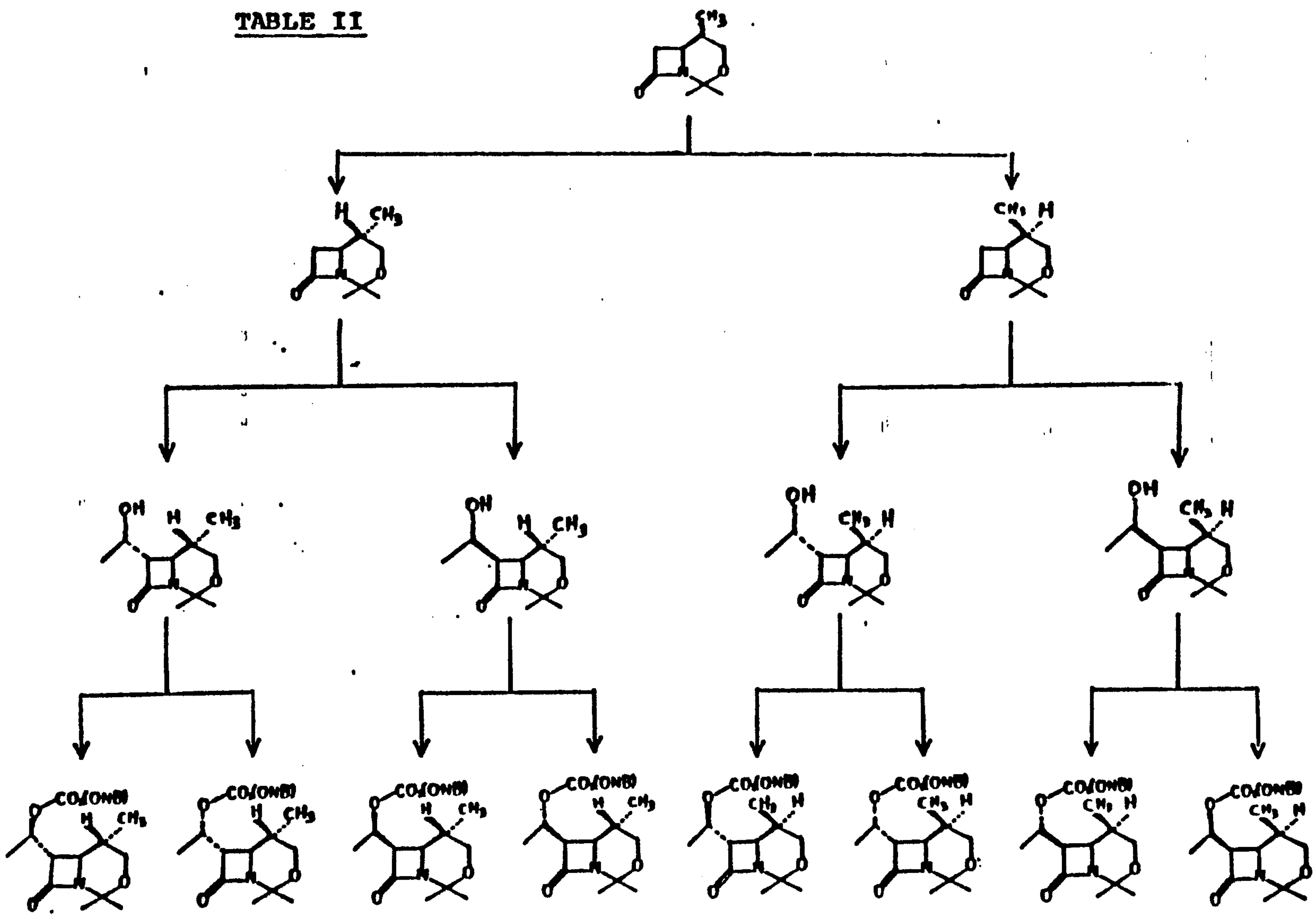

(ONB=o-nitrobenzyl)

Step Ga

Preparation of R-trans-9'β:

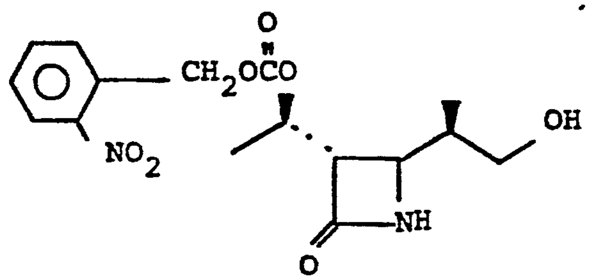

R-trans-9'β

8-Oxo-3-oxa-5β,2,2-trimethyl-7α-(1R-o-nitrobenzyloxycarbonyloxyethyl)-1-azabicylco-[4.2.0] octane (R-trans-7"β) (2.1 g) is dissolved in 4 ml trifluoroacetic acid and 4 ml water at room temperature and the mixture is stirred for 10 minutes. The resulting homogeneous solution is slowly poured into a vigorously stirred saturated solution of potasssium bicarbonate (30 ml) in a 200-ml beaker. The mixture is extracted with methylene chloride (200 ml). The organic layer is separated, dried over $Na_2SO_4$ and allowed to evaporate *in vacuo* to give crude product 9' which is purified by a silica gel column eluting with 40% ethylacetate/cycylhexane to afford product R-trans-9'β as an oil.

NMR (300 MHz, $CDCl_3$): 0.98 (d), 1.28 (d), 2.85 (m), 3.20 (q), 3.62 (m), 5.12 (m), 5.57 (q), 6.40 (s), 7.53 (t), 7.66 (m) and 8.14 (d).

Step Gb

Following the procedure of Step Ga, except systematically replacing the starting material with the other isomers, the other isomeric products are obtained (Table III). Equivalently R·may be the para- isomer.

TABLE III

R= 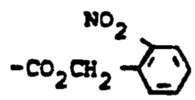 ; or 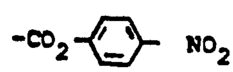

| Starting material | | Product | |
|---|---|---|---|
| 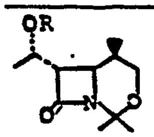 | S-trans-7"β | 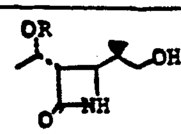 | S-trans-9'β |
| 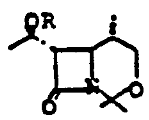 | R-trans-7"α | 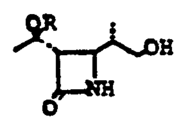 | R-trans-9'α |
| 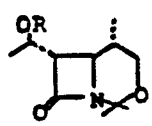 | S-trans-7"α | 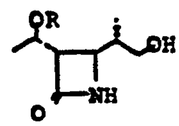 | S-trans-9'α |
| 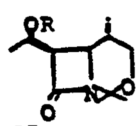 | R-cis-7"α | 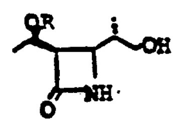 | R-cis-9'α |
| 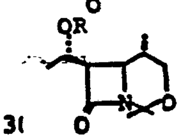 | S-cis-7"α | 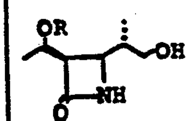 | S-cis-9'α |
| 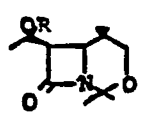 | R-cis-7"β | 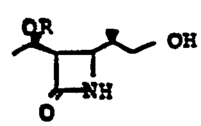 | R-cis-9'β |
| 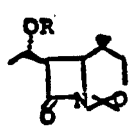 | S-cis-7"β | 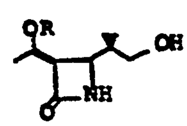 | S-cis-9'β |

Steps H-K

Steps H, I, J and K complement Steps E, Fa-d, and G for the preparation of 3-(1-p(and o)-nitrobenzylcarbonyldioxyethyl)-4-(1-methyl-2-hydroxyethyl) azetidinone

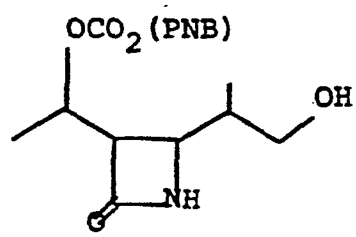

Step H

Preparation of 1-(2-Tetrahydropyranyl)-4-[1-metnyl-2-(2-tetrahydropyranyl)oxyethyl]-2-azetidinone

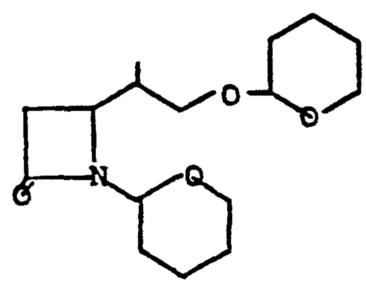

Under nitrogen and at 25°C, a solution of 4-(1-methyl-2-hydroxyethyl)-2-azetidinone (62 mg), in 0.5 ml of anhydrous p-dioxane is treated with 2,3-dihydropyran (.98 ml) and p-toluenesulfonic acid monohydrate (19 mg). The resulting solution is stirred for a period of 60 minutes and then partitioned between 10 ml of .5M pH 7 phosphate buffer and 10 ml of ethyl acetate. The aqueous phase

is extracted a second time with ethyl acetate. The combined ethyl acetate solutions are washed with brine, dried over magnesium sulfate and filtered. The filtrate is evaporated under reduced pressure to give 216 mg of crude product. Purification by preparative thick-layer chromatography developing with ethyl acetate, gives 80 mg of 1-(2-tetrahydropyranyl)-4-[1-methyl-2-(2-tetrahydropyranyl)-oxyethyl]-2-azetidinone as an oil.

Step I

Preparation of Cis and Trans-1-(2-tetrahydropyranyl)-3-(1-hydroxyethyl)-4-[1-methyl-2-(2-tetrahydropyranyl)-oxyethyl]-2-azetidinone

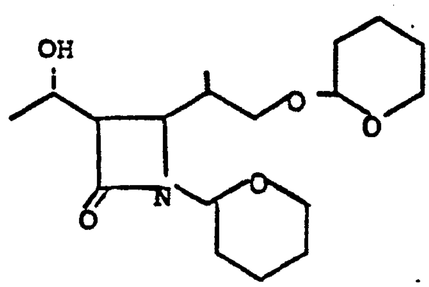

Following the procedure described for the preparation of 8-oxo-2,2,5-trimethyl-7α and β-(1-hydroxyethyl)-3-oxa-1-azabicyclo[4.2.0]octane from 8-oxo-2,2,5-trimethyl-3-oxa-1-azabicyclo[4.2.0]octane and using 1-(2-tetrahydropyranyl)-4-[1-methyl-2-(2-tetrahydropyranyl)oxyethyl]-2-azetidinone one obtains a diastereomeric mixture of both cis and trans -1-(2-tetrahydropyranyl)-3-(1-hydroxyethyl)-4-[1-methyl-2-(2-tetrahydropyranyl)oxyethyl]-2-azetidinone.

Step J

Preparation of Cis and Trans-1-(2-tetrahydropyranyl)-3-(1-p-nitrobenzylcarbonyldioxyethyl)-4-[1-methyl-2-(2-tetrahydropyranyl)oxyethyl]-2-azetidinone

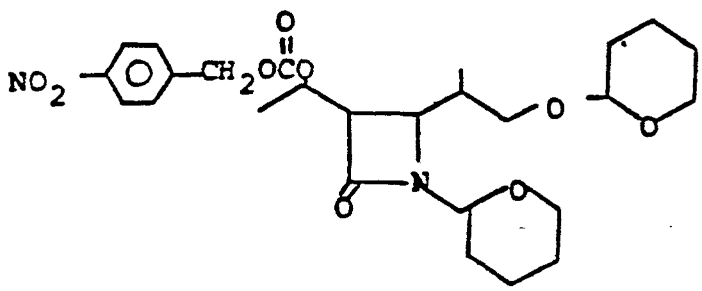

Following the procedure described for the preparation of 8-oxo-2,2,5-trimethyl-7α-(1-p-nitrobenzylcarbonyldioxyethyl)-3-oxa-1-azabicylco[4.2.0]octane from 8-oxo-2,2,5-trimethyl-7α-(1-hydroxyethyl)-3-oxa-1-azabicyclo[4.2.0]octane and using trans-1-(2-tetrahydropyranyl)-3-(1-hydroxyethyl)-4-[1-methyl-2-(2-tetrahydropyranyl)oxyethyl]-2-azetidinone there is obtained trans-1-(2-tetrahydropyranyl)-3-(1-p-nitrobenzylcarbonyldioxyethyl)-4-[1-methyl-2-(2-tetrahydropyranyl)oxyethyl]-2-azetidinone. The cis diastereoisomers are obtained in an analogous manner.

Step K

Preparation of Cis and Trans-3-(1-p-nitrobenzyl-carbonyldioxyethyl)-4-(1-methyl-2-hydroxyethyl)-2-azetidinone

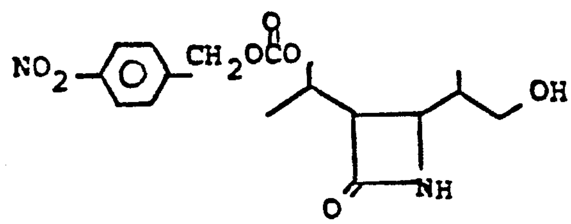

A solution of trans-1-(2-tetrahydropyranyl)-3-(1-p-nitrobenzylcarbonyldioxyethyl-4-[1-methyl-2-(2-tetrahydropyranyl)oxyethyl]-2-azetidinone in methanol at 25°C is treated with .1 molar equivalent of p-toluenesulfonic acid monohydrate. The solution is stirred for a period of 2 hours and then neutralized with 1M pH 7 phosphate buffer. The product is extracted into ethyl acetate. The ethyl acetate solution is washed with brine, dried over magnesium sulfate and filtered. The filtrate is evaporated under reduced pressure to give trans-3-(1-p-nitrobenzyloxycarbonyloxyethyl)-4-(1-methyl-2-hydroxyethyl)-2-azetidinone. The cis diastereoisomers are obtained in an analogous manner.

Step L

Preparation of 12

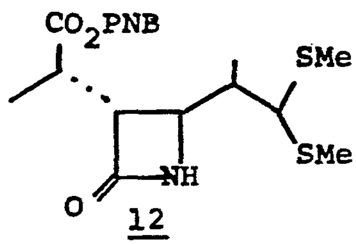

12

PNB = 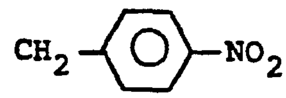

To 6.75 anhydrous pyridine (83.9 mmole) in 350 ml anhydrous acetonitrile is added 4.05 g anhydrous powdered chromium trioxide (mw = 100; 40.5 mmole). After stirring at room temperature (25°C) for 30 minutes, 9.6 g dried Supercell is added and stirring is continued for 5 additional minutes. A solution of 3.21 g trans-3-(1-p-nitrobenzylxoycarbonyloxyethyl)-4-(1-methyl-2-hydroxy-ethyl)-2-azetidinone in 30 ml anhydrous acetonitrile is added all at once. The reaction mixture is stirred under anhydrous conditions at room temperature (25°C) for one hour. Addition of 9.6 g $NaHSO_3$ is followed by 5 minutes of stirring after which the reaction mixture is filtered through a mixed packed bed of 40 g silica gel and 40 g anhydrous magnesium sulfate. The bed is washed repeatedly with acetonitrile (total volume of filtrate 600 ml). The filtrate is concentration under a $N_2$ stream to 130 ml total volume. To this solution containing crude aldehyde at 0°C under $N_2$ is added 9.64 g methylmercaptane. To the stirred reaction mixture is mixture is added 8.0 ml boron

triflfuoride ethereate (63.4 mmole). After 1.5 hours at 0°C the reaction mixture is poured into a stirred ice-cold mixture of 69 g $K_2HPO_4$ - 500 ml $H_2O$ and 700 ml ethyl acetate (EA). The layers are separated, and the aqueous one is saturated with NaCl and re-extracted with additional EA. The combined organic layers are washed twice with brine, dried over anhydrous $MgSO_4$ and filtered. The filtrate is concentrated under a $N_2$ stream and then pumped on high vacuum to give crude 12.

The material is chromatographed on 450 g silica gel (column height = 48 cm; diameter = 5.5 cm) packed and applied in $CHCl_3$ and eluted with increasing percentages of MeOH in $CHCl_3$ (0-4% MeOH/$CHCl_3$). Those fractions containing the desired product are combined, concentrated under a $N_2$ stream; and pumped on high vacuum to give 12.

Step N

Preparation of 14

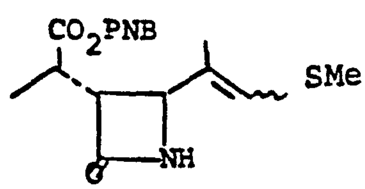

14

To 14.2 ml pentane (dried over 4A Linde molecular sieves) is added 0.5 ml $Br_2$. To 5 g of

12 in 58 ml tetrahydrofuran (THF) (freshly distilled from lithium aluminum hydride (LAH) and 65 ml $Et_2O$ (dried over 3A 1/16" Linde molecular sieves) at 0°C under $N_2$ with stirring is added dropwise 10 ml of the above 0.66 M $Br_2$ solution. After 10 minutes at 0°C, 0.67 ml cyclohexene is added. After 5 minutes at 0°C, 1.7 ml triethylamine is added immediately followed by 40 ml ice-cold dimethylformamide (DMF) (distilled from anhydrous $CaSO_4$ at 40 mm and stored over 4A Linde molecular sieves). The ice bath is removed, and stirring is continued for 2-1/4 hours at room temperature. The reaction mixture is poured into a stirred ice-cold mixture of 12.6 ml $1MKH_2PO_4$ 160 ml $H_2O$ - 500 ml (EA). After separation of the layers, the aqueous one is saturated with sodium chloride and re-extracted with EA. The combined organic layers are extracted once with brine, dried over anhydrous $MgSO_4$, filtered and concentrated under a $N_2$ stream followed by pumping under high vacuum to provide crude 14.

The material is chromatgraphed on 250 g silica gel (height = 45 cm; diameter = 4.5 cm) packed and applied in $CHCl_3$ and eluted with increasing percentages of MeOH in $CHCl_3$ (0-3% MeOH/$CHCl_3$). Those fractions containing clean product are combined, concentrated under a $N_2$ stream, and pumped on high vacuum to give 14.

Step O

Preparation of 15

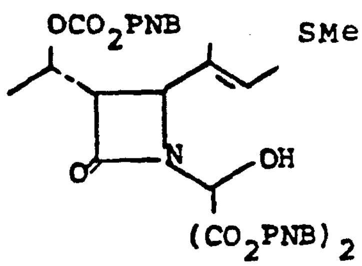

15

To a stirred solution of 2.48 g di(p-nitrobenzyl)-ketomalonate (Step P) in 400 ml hot anhydrous toluene is added a solution of 2.52 g of 14 in 20 ml THF (distilled from LAH) and 40 ml anhydrous toluene. After some of the solvent is boiled off, additional anhydrous toluene is added, and the azeodrying process is repeated three times. The solution is then refluxed under $N_2$ for 30 minutes. Additional tolune is then allowed to boil off yet the volume is not allowed to diminish so much that precipitation occurs. Total heating time is approximately 2-1/2 hours. The clear yellow reaction mixture is removed from the oil bath and placed under a stream of $N_2$. After concentration to a oil, the residue is dissolved in $CH_2Cl_2$, dried over anhydrous $MgSO_4$, filtered, and concentrated under a $N_2$ stream to give crude 15.

The material is chromatographed on 250 g silica gel packed and applied in $CHCl_3$ (height = 43 cm; diameter = 4.5 cm). Elution with 500 ml 0.5% MeOH/$CHCl_3$ is followed by continued elution with 1%

$MeOH/CHCl_3$ for the remainder of the chromatography. After the emergence of excess reagent, those fractions containing pure 15 are combined, concentrated under a $N_2$ stream and then on high vacuum to give 15.

Step P

Preparation of di-p-Nitrobenzyl Ketomalonate

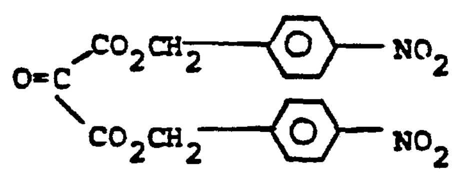

A mixture of 100 g p-nitrobenzyl bromide (0.46 mole), 28.6 g malonic acid (0.275 mole) and 750 ml ethanol) (EtOH) is stirred and warmed on the steam bath until solution is achieved. A solution of 33 g KOH in 200 ml of water is added carefully with swirling. An additional 200 ml of water is added, and the two-phase system is refluxed for 1.8 hours. The lighter color homogeneous solution is cooled in ice for 1 hour and the crude product isolated by filtration, washed twice with a minimum of cold EtOH, and dried by pulling dry $N_2$ through the cake; 33.7 g of solid is obtained. If, curing the refluxing stage the reaction mixture is slowly concentrated to ca. half volume by allowing refluxing solvent to distill off, the crude product yield rises to 77 g.

The material is recrystallized from methanol to give pure di-p-nitrobenzyl malonate.

A mixture of 23.4 od di-p-nitrobenzyl malonate 10 g $SeO_2$, and 30-40 ml of xylene is stirred in a flask immersed in an oil bath. The bath temperature is raised over 1 hour to 130-135°, most of the insoluble residue is black Se°. The mixture is cooled, $MgSO_4$ is added to remove the water, and Celite is added to aid in filtration. The mixture is filtered through Celite and the cake washed with xylene and a small portion of EtOAc. Final volume: 60 ml. A 100 g column of Baker silica gel is prepared in benzene and 10 ml of filtrate applied, then eluted with increasing amount of EtOAc in benzene, 500 ml fractions being collected. After one 2% ethyl acetate (EtOAc)/OH, and two 10% EtOAc/OH fractions, the third 10% and first 20% EtOAc/OH provide the bulk of the product (1.6 g from 10 ml filtrate) and judged by tlc (20% EtOAc/$CHCl_3$; silica gel GF). Recrystallization from benzene, (1 g in ca. 50 ml concentrated to 1/3 volume and "spiked" with 1 ml of $H_2O$ saturated benzene): provides di-p-nitrobenzyl ketomalonate.

<u>Step Q</u>

<u>Preparation of 17</u>:

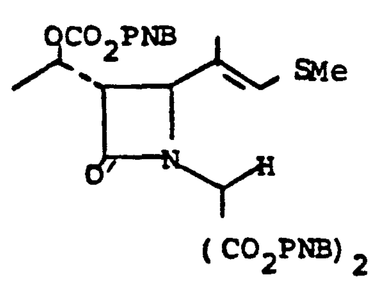

A solution of 1.468 g of 15 in $CH_2Cl_2$ is dried over anhydrous $MgSO_4$, filtered, and concentrated under a $N_2$ steam, and dried further under high vacuum just prior to the following reaction. To a solution of 15 in 24 ml THF (freshly distilled from LAH) at -20°C is added 0.206 ml anhydrous pyridine. With stirring under $N_2$, 294 mg of freshly distilled thionyl chloride in 5 ml THF is added dropwise. The reaction mixture is stirred for 10 minutes at -20°C., then 1/2 hour at 0°C and finally 1 hour at 25°C. The pyridine hydrochloride is filtered under $N_2$ and washed with 20 ml THF. The filtrate is concentrated under $N_2$ stream followed by pumping on high vacuum.

To this freshly prepared chloro compound is added witn stirring a freshly shaken suspension of 678 mg tributylphosphine in 36.5 ml 9:1 DMF - $H_2O$ followed by 294 mg $K_2HPO_4$. The reaction mixture is stirred at 25°C., for 35 minutes. After dilution with 120 ml EA and 60 ml brine, the layers are separated, and the aqueous one is extracted two times with EA. The combined organic layers are washed one time with brine, dried over anhydrous $MgSO_4$, filtered an concentrated under a $N_2$ stream followed by pumping on high vacuum to give crude 17.

The material is chromatographed on 100 g silica gel (height = 28.5 cm; d = 4 cm) packed and applied in $CHCl_3$ and eluted with 0.5% MeOH in $CHCl_3$. Those fractions containing clean product are combined, concentrated under a $N_2$ stream and then on high vacuum to give 17. (EA = ethyl acetate.)

Step R

Preparation of 19:

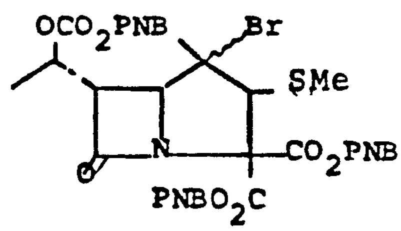

To 8.5 ml pentane (dried over 4A Linde molecular sieves ) is added 0.2 ml $Br_2$. To 0.706 g of 17 in 18 ml THF (freshly distilled from LAH) and 5.7 ml $Et_2O$ (dried over 3A 1/16" Linde molecular sieves) at 0°C under $N_2$ with stirring is added dropwise 1.8 ml of the above 0.45M $Br_2$ solution (0.81 mmole) to give dibromide 18. After 15 minutes at 0°C., 0.42 ml triethylamine is added immediately followed by 10.5 ml ice-cold DMF (distilled from $CaSO_4$ at 40 mm and stored over 4A Linde molecular sieves). The ice-bath is removed, and stirring at room temperature is continued for 2 hours. The reaction mixture is poured into a stirred, ice-cold mixture of 2.1 ml 1M $KH_2PO_4$ - 70 ml $H_2O$ - 100 ml EA. The layers are separated, and the aqueous one is saturated with NaCl and re-extracted with EA. The combined organic layers are washed once with brine, dried over anhydrous $MgSO_4$, and filtered. The filtrate is concentrated under a $N_2$ stream and then pumped on high vacuum to give crude 19.

The material is chromatographed on 60 g silica gel (diameter = 2.8 cm) packed and applied in

$CHCl_3$ and is eluted with 100 ml - 2% EA/$CHCl_3$; 100 ml - 4% EA/$CHCl_3$ and then 5% EA/$CHCl_3$ for the remainder of the chromatography. Tne fractions containing pure 19 are combined, concentrated under a $N_2$ stream, and pumped on high vacuum to give 19.

Step S:

Preparation of 20:

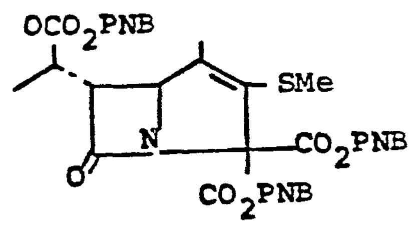

To 29 mg anhydrous silver fluoride is added a solution of 146 mg of 19 in 3.5 ml anhydrous pyridine. The stoppered reaction mixture is stirred at room temperature in a dark for one hour and then pouredinto 20 ml cold water - 30 ml EA. After separation of the layers, the aqueous one is extracted two times with EA and one time with $CHCl_3$. Each organic layers is extracted one time with $H_2O$ and one time with brine. The combined organic layers are dried over anhydrous $MgSO_4$, filtered, and concentrated under a $N_2$ stream followed by pumping on high vacuum to give crude 20.

Preparative thin layer chromatography (eluant = 40% acetone/hexane; repeated extraction of

desired u.v. band with a large volume of $CHCl_3$) yields slightly contaminated 20. Re-chromatographing on silica using EA in $CHCl_3$ as an eluting system gives pure 20.

Step T:

Preparation of 21:

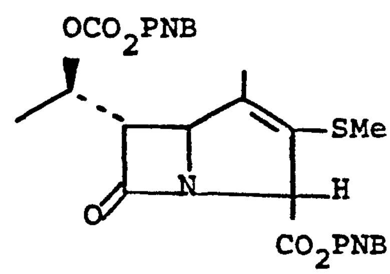

A solution of 77 mg of 20 in 0.9 ml collidine (distilled from powdered KOH) is added to 13.4 mg anhydrous LiI (dried for few hours at 100%C over $P_2O_5$ under vacuum). With stirring under $N_2$, the reaction mixture is heated in an oil bath at 120°C. After a total of 30 minutes, the reaction mixture is cooled to 25°C., diluted with $CH_2Cl_2$, and transferred to a round bottom flask for concentration under a $N_2$ stream and then on high vacuum. Partitioning the residue between EA-$H_2O$ and 1 ml 1M $KH_2PO_4$ is followed by extraction of the aqueous layer two additional times with EA and one time with $CHCl_3$. Each organic layer is then backwashed with brine. The combined organic layers are dried over anhydrous $MgSO_4$ filtered, concentrated under a $N_2$ stream and then on high vacuum to give crude 21.

Preparative thin layer chromatography on silica gel yields 21.

Step U:

Preparation of 22:

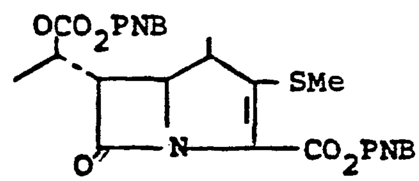

To ~~49 mg of~~ 21 in 0.7 ml DMSO (distilled from $CaH_2$ at 8 mm and stored over 4A Linde molecular sieves) is added 100 µl diisopropylamine (distilled from NaH under $N_2$ and stored over 4A Linde molecular sieves). The stoppered reaction mixture is stirred for a few minutes and then allowed to stand for 2 hours. The amine and most of the DMSO are then concentrated off under high vacuum with no external heating. The residue is passed quickly through a column of silica gel (packed, applied and eluted with EA) to remove residual DMSO. After concentration under a $N_2$ stream of all fractions having u.v. absorbance, the material is chromatographed on a thin layer silica gel plate (eluant = 50% EA/$CHCl_3$; repeated extraction of desired u.v. bands with a large volume of chloroform) to give 22.

Step V:

Preparation of I:

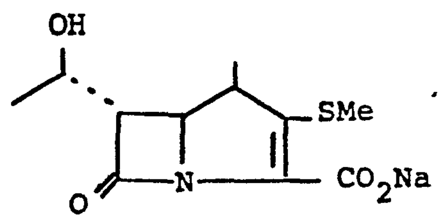

To 5.2 mg 22 is added 0.60 ml dioxane, 0.05 ml ethanol, 0.35 ml deionized water and 0.01 ml of 1.0M $Na_2HPO_4$. To the resultant clear solution is added 5 mg of 10% Pd/C. The suspension is flushed with $N_2$, then 5-6 times alternately with 50 psi $H_2$ atmosphere for 30-40 minutes. After centrifugation, the Pd/C is washed and centrifuged 2-3X with 0.5 ml portions of deionized water. The combined centrifugates are extracted 5x1 - 2 ml ether. Residual ether is removed under vacuum and the aqueous solution is chromatographed on an XAD-2 column (20 X 140 mm) which is eluted with water to give the desired product I.

Step X:

Esterification of I:

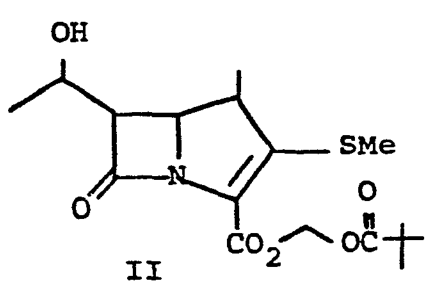

II

The sodium salt I (100 mg) suspended in DMF (1 ml) is treated with pivaloyloxymethyl chloride (2 eq) at room temperature for 3 hours. The mixture is then diluted with ethyl acetate and washed with water. The organic layer is separated, dried over $Na_2SO_4$ then chromatographed by a silica gel column eluting with 50% ethylacetate/hexane to give ester II.

EXAMPLE 2

Step A:

Preparation of 3-methyl-1,4-pentadiene

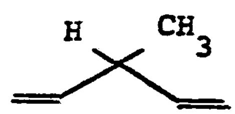

Procedure a

3-methylglutaric acid (obtained from Aldrich Chemical Company) (one mole), is refluxed for 2 hours with thionyl chloride (68%) excess). After removal of excess thionyl chloride, absolute ethanol (109% excess) is added slowly. The mixture is refluxed for 3 hours then distilled to collect the product, diethyl β-methylglutarate (98% yield).

To a suspension of lithium alumium hydride (24 g) in ether (860 ml) is added dropwise with rapid

stirring a solution of diethyl β-methyl-glutarate (124 g in 250 ml ether). The mixture is refluxed for 6 hours, then cooled to room temperature. Water (25 ml) is added slowly. The mixture is then titrated with 10% NaOH until a clear organic layer is obtained. The organic layer is separated, dried over anhydrous sodium sulfate then evaporated in vacuo to give the resulting diol as an oil. The 3-methyl-1.5-pentanediol (0.5 mole)is treated with thionyl chloride (1.05 mole) at reflux for 3 hours. After removal of excess thionyl chloride in vacuo, the 3-methyl-1,5-dichloropentane is obtained.

3-methyl-1.5-dichloropentane (41 g) is added dropwise at 170°C to a mixture of 48 g of sodium hydroxide and 40 g of polyethylene glycol tetramer and the mixture is distilled to give 3-methyl-1,4-pentadiene.

Step B:

Preparation of 4-(1-methyl-pro-2-enyl)azetidin-2-one

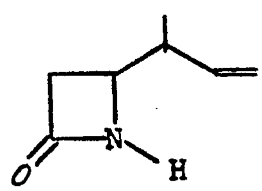

In a sealed tube, 3-methyl-1,4-pentadiene (9.6 g) and chlorosulfonyl isocyanate (14.2 g) are allowed to stand at room temperature for 6 days. The

resulting mixture is diluted with methylene chloride and added slowly to a stirred aqueous solution which contains 20 g of $Na_2SO_3$ and 50 g of $K_2HPO_4$ at 0-5°C for 30 minutes. The organic layer is separated and dried over $Mg_2SO_4$. After evaporation, the crude product is chromatographed on silica gel GF eluting with EtOAc to give 2.

Step C:

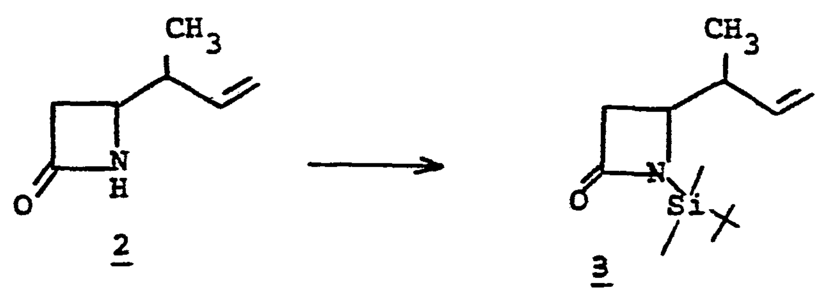

2

3

t-Butyldimethylchlorosilane (7.51 g) is added in one portion to an ice-cold, stirred solution of 4-(1-methyl-prop-2-enyl)-azetidin-2-one (6.54 g) and triethylamine (12 ml) in anhydrous dimethylformamide (100 ml). The reaction mixture is stirred at 0-5°C for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3 x 50 ml), and brine, dried with magnesium sulfate, filtered and evaporated under vacuum to provide a crude product which is purified by chromatography on silica gel (20% ether in petroleum ether) to yield 3.

Step D:

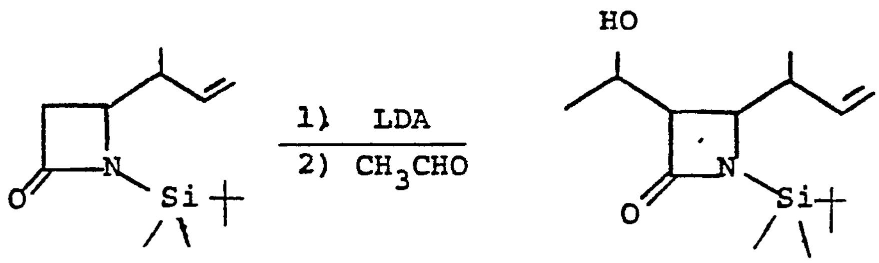


3 4

n-Butyllithium in hexane (26.25 mmol) is added slowly by syringe to a solution of diisopropylamine (26.25 mmol) in anhydrous tetrahydrofuran (100 ml) at -78°C. The resulting solution is stirred for 15 min. prior to the addition of a solution of 4 (25.0 mmol) in anhydrous tetrahydrofuran (25 ml). After stirring for 15 minutes at -78°C, acetaldehyde (75 mmol) is added by syringe and the resulting solution is stirred at -78°C for 5 minutes. Saturated aqueous ammoniuim chloride solution (15 ml) is added by syringe and the reaction mixture is allowed to warm to room temperature, then diluted with ether (250 ml) and washed with 2.5N hydrochloric acid solution (2 x 50 ml), water (100 ml) and brine and dried over magnesium sulfate. Solvents are removed *in vacuo* and the residue is chromatographed on silica gel (1:1, ether:petroleum ether) to give the expected product 4.

Step E:

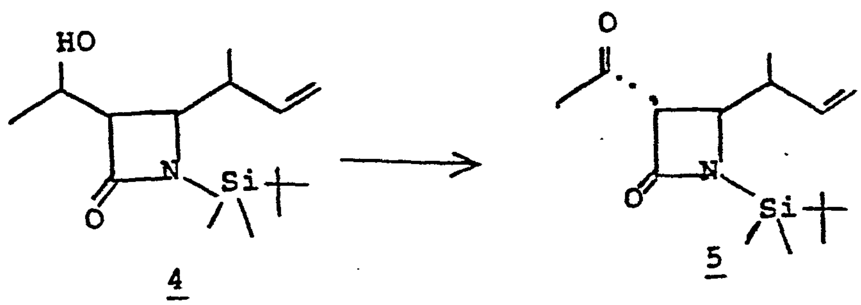

4 5

A. Trifluoroacetic anhydride (7.5 mmol) is added dropwise by syringe to a solution of dimethylsulfoxide (10 mmol) in anhydrous methylene chloride (15 ml) at -78°C. The resulting mixture is stirred at -78°C for 20 minutes. A solution of 4 (5.0 mmol) in methylene chloride (15 ml) is added by syringe and the cooling bath is removed. After an additional 1 hour, the reaction mixture is diluted with methylene chloride (100 ml), washed with water (50 ml) and brine and dried over magnesium sulfate. Removal of solvents *in vacuo* yields crude product which is chromatographed on silica gel (2:1, petroleum ether:ether) to yield 5.

Step F:

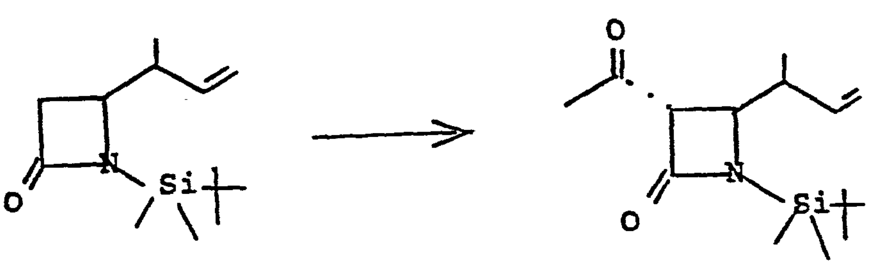

3 5

B. n-Butyllithium in hexane (4.10 mmol) is added by syringe to a solution of diisopropylamine (4.10 mmol) in anhydrous tetrahydrofuran (16 ml) at -78°C. The resulting solution is stirred at -78°C for 15 minutes prior to the addition of a solution of 1-(t-butyl-dimethylsilyl)-4-(1-methyl-prop-2-enyl)-azetidin-2-one 3 (2.0 mmol) in anhydrous tetrahydrofuran (2 ml). After an additional 15 minutes at -78°C, the reaction mixture is added *via* a Teflon tube to a mixture of N-acetylimidazole (4.1 mmol) in anhydrous tetrahydrofuran (16 ml) at -78°C. the resulting yellow reaction mixture is stirred at -78°C for 15 minutes, then quenched by addition of saturated aqueous ammonium chloride solution (10 ml). The reaction mixture is diluted with ether (100 ml) and washed with 2.5N hydrochloric acid solution (25 ml) water (25 ml) and brine. The organic phase is dried over magnesium sulfate and concentrated *in vacuo* to yield a crude product. This material is chromatographed on silica gel (2:1 petroleum ether: ether) to yield 5.

Step G:

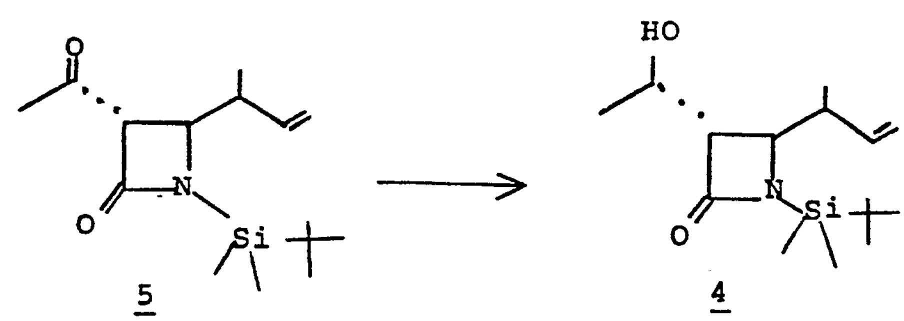

5 4

K-Selectride (potassium tri-(sec)-butylborohydride) in tetrahydrofuran (4.8 mmol) is added by syringe to

a mixture of potassium iodide (2.0 mmol) and 5 (2.0 mmol) in anhydrous ether (20 ml) at room temperature. The resulting mixture is stirred at room temperature for 2.5 hours, then quenched by addition of glacial acetic acid (9.6 mmol). The resulting mixture is diluted with ethylacetate (100 ml) and filtered through celite. Removal of solvents *in vacuo* gives crude product which is chromatographed on silica gel (1:1 ether: petroleum ether) to yield 1.90 g (95%) of 4.

Step H:

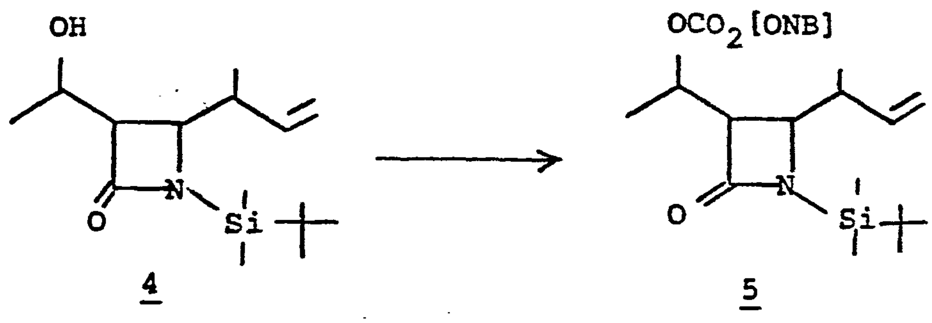

4 5

ONB = o-nitrobenzyl

Under anhydrous conditions at 0°C a solution of 6 (3.50 g) in 60 ml methylene chloride is treated with 4-dimethylaminopyridine (3.32 g) and o-nitrobenzylchloroformate (5.88 g). The mixture is allowed to warm to room temperature and stirred for 1 hour. the resulting mixture is washed with 0.1N HCl, water, brine and water. The organic layer is separated, dried over $Na_2SO_4$ and allowed to evaporate *in vacuo* to give crude products. The crude products, dissolved in 20 ml ether and chilled at -5°C, give the o-nitrobenzyl alcohol (0.5 g) which is

separated by filtration. Purification by HPLC (silica gel) eluting with 40% ethylacetate/cyclohexane to gives 6.

Step I:

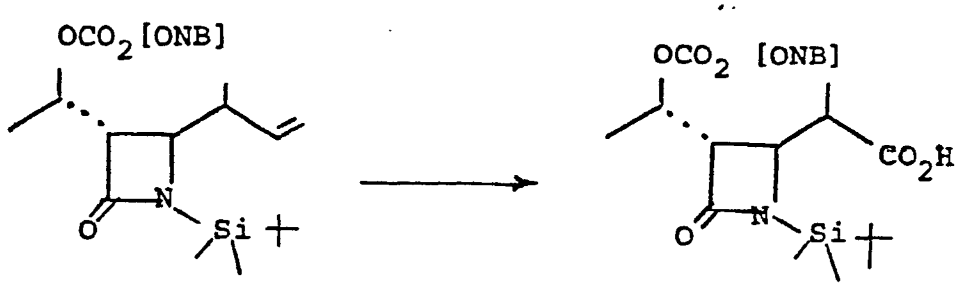

6 7

A solution of 7 (3.0 mmol) in dry methylene chloride (30 ml) is cooled to -78°C (dry ice-acetone) and a stream of ozone is bubbled through until the reaction mixture becomes blue. The ozone flow is then stopped and the reaction is purged by bubbling through nitrogen until the blue color disappears. Solid m-chloroperbenzoic acid (3.0 mmol) is added and the cold bath is removed. When the reaction mixture reaches room temperature, the flask is fitted with a reflux condenser and the mixture is heated at reflux for three days. Removal of solvents in vacuo gives crude product which is chromatographed on silica gel (2% glacial acetic acid in methylene chloride) to 7.

Step J:

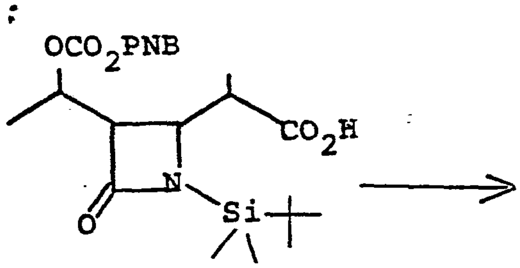

7 8

The acid 7 (1.0 mmol) is hydrogenated in 30 ml ethyl acetate under 1 atm $H_2$ in the presence of 0.1 mmol of 10% Pd/C at room temperature for 30 minutes. The mixture is filtered from catalyst. The filtrate is evaporated in vacuo to give 8.

Step K:

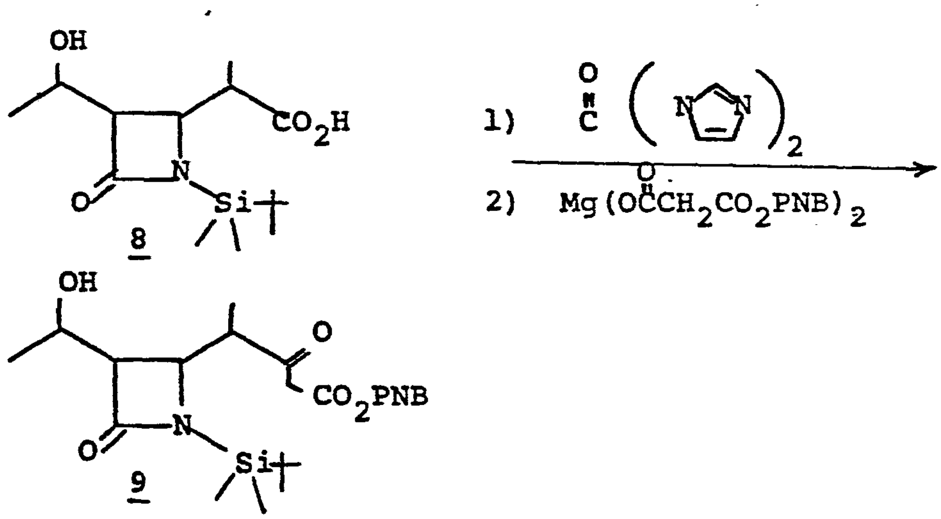


8

9

1,1'-Carbonyldiimidazole (1.10 mmol) is added in one portion to a solution of 8 (1.0 mmol) in anhydrous tetrahydrofuran (5 ml) at room temperature. The resulting solution is stirred at room temperature for

6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hour, then the tetrahydrofuran is removed at the pump and the residue is triturated with ether to yield the magnesium salt. This magnesium salt is then added to the first reaction flask and the resulting mixture is stirred at room temperature for 18 hours. The reaction mixture is then poured into 50 ml of ether, washed with 0.5N hydrochloric acid solution (20 ml), water (20 ml), saturated aqueous sodium bicarbonate solution (20 ml), brine and dried over magnesium sulfate. Removal of solvents *in vacuo* gives crude product which is chromatographed on silica gel (ether) to yield 9.

Step L:

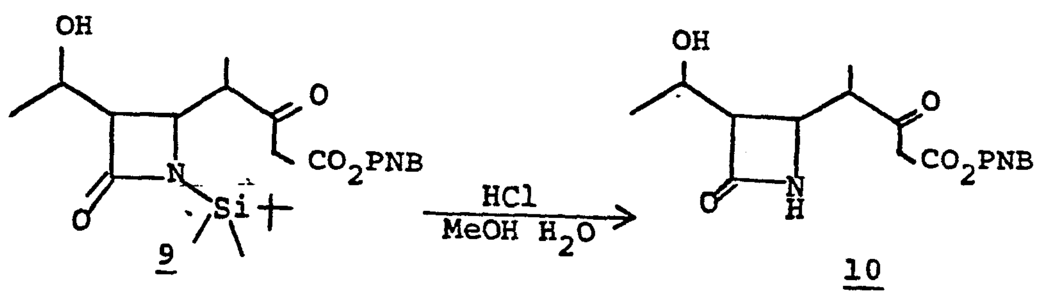


9 → 10

A solution of 9 (1.0 mmol) in 20 ml of 9:1 (v/v) methanol-water is cooled to 0°C. Concentrated hydrochloric acid (0.34 ml) is added and the resulting solution is stirred at 0°C for 15 minutes, then allowed to warm to room temperature. After 2.5 hours, at room temperature the reaction mixture is

diluted with ethyl acetate (25 ml), washed with water (10 ml) and brine, dried over magnesium sulfate and concentrated in vacuo to yield 10.

Step M:

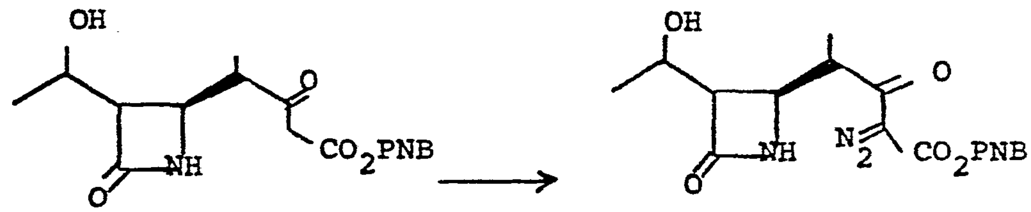

10 11

Triethylamine (263 mg) is added by syringe to a mixture of 10 (253 mg) and p-toluenesulfonylazide (196 mg) in dry acetonitrile (6 ml) at 0°C. The cooling bath is removed and the reaction mixture is stirred at room temperature for 1 hour. The mixture is then diluted with ethyl acetate (50 ml) and filtered. The filtrate is concentrated in vacuo and the residue is chromatographed on a short silica gel column (ethyl acetate) to yield 11.

Step N:

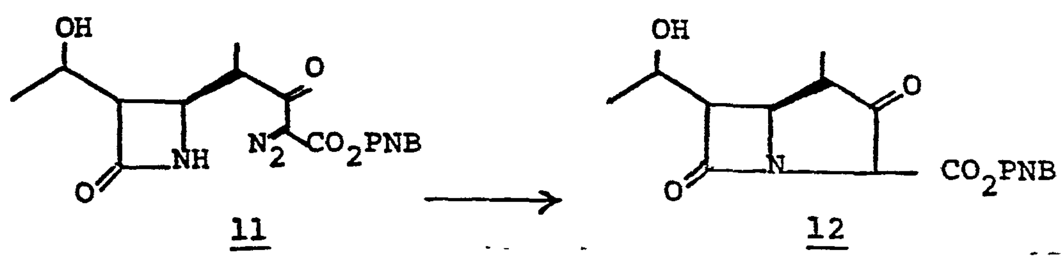

11 12

A suspension of 11 (56.4 mg) and rhodium (II) acetate (0.1 mg) in cyclohexane (3 ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield 12.

Step O:

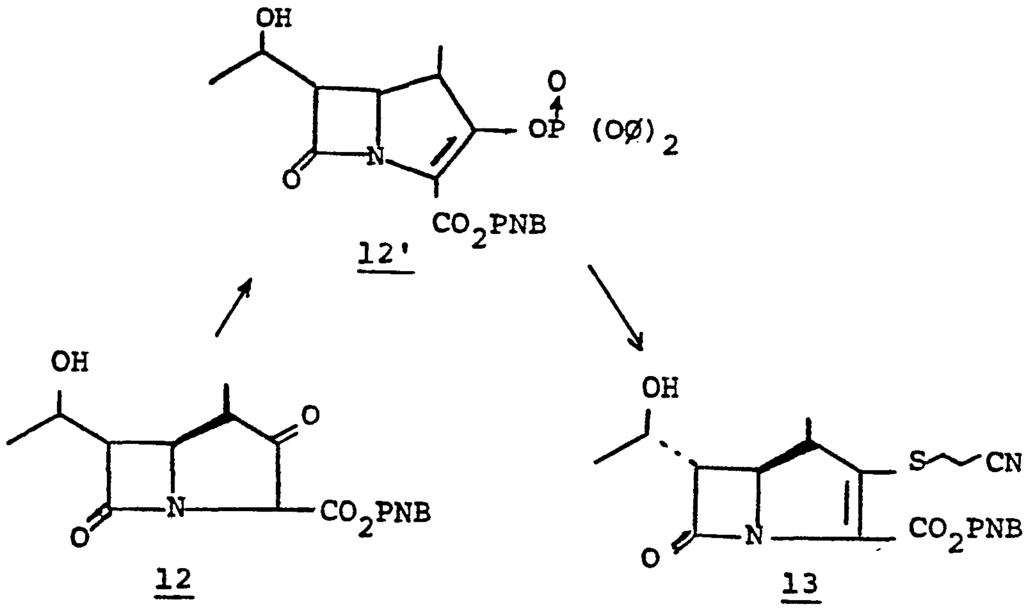

12'

12

13

The starting material 12 (51 mg) is dissolved in acetonitrile (3 ml) and the resulting solution is cooled at 0°C. Diisopropylethylamine (22 mg) is added by syringe and the resulting solution is stirred at 0°C for 1 minute prior to the addition of a solution of diphenylchlorophosphate (51 mg) in dry acetonitrile (1 ml). The resulting solution is stirred at 0°C for 1 hour to provide intermediate 12', then cooled to -25°C. Diisopropylethylamine (80.5 mg) is added by syringe followed shortly thereafter

by a solution of cyanoethylthiol (40 mg) in 1 ml of dry acetonitrile. The reaction mixture is then stored in a refrigerator for 70 hours. The mixture is diluted with 25 ml of ethyl acetate washed with brine and dried over magnesium sulfate. Solvents are removed in vacuo to yield crude product which is chromatographed on a silica gel plate to yield 13.

Step P:

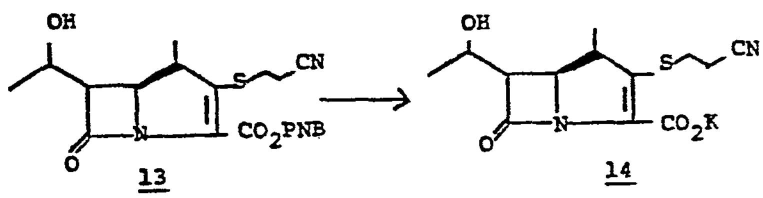

13 14

A mixture of 13 (10 mg) and 10% Pd/C in tetrahydrofuran (2 ml), 0.1M dipotassium hydrogen phosphate solution (1.4 ml) and 2-propanol (0.2 ml) is hydrogenated at 40 psi on the Par shaker for 30 minutes. The mixture is then filtered and the catalyst is washed with water. The combined filtrate and washings are extracted with ethyl acetate-ethyl ether then concentrated to 3 ml and lyophilized to give 14.

Step Q:

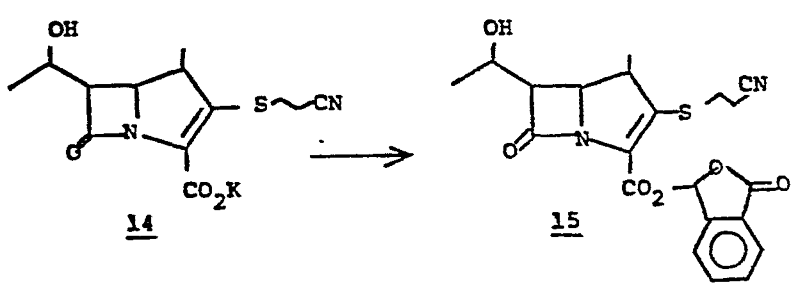

14 15

The potassium salt (100 mg) is treated with chlorophathilide (2 eq) in DMF (1 ml) at room temperature for 3 hours. The mixture is diluted with ethylacetate then washed with water. The organic layer is separated, dried over $Na_2SO_4$ and chromatographed on silica gel column to give desirable ester 15.

EXAMPLE 3

Preparation of Chiral Carbapenem esters:

(5R,6S,8R)-1ß-methyl-2-ethylthio-6-(1-hydroxyethyl)-1-carbapen-2-em-3-carboxylic acid (5-methyl-2-oxo-1,3-dioxolen-4-yl)methyl ester

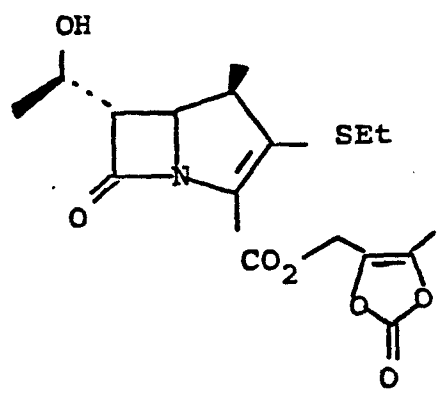

Step A:

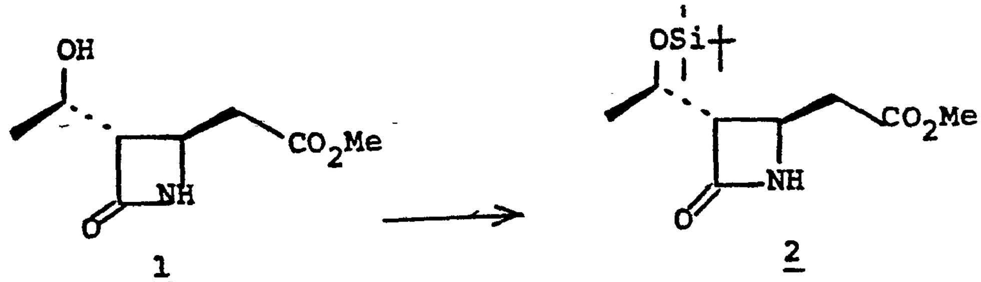

1 2

The chiral azetidinone 1 (374 mg) in DMF is treated with imidazole (688 mg) and t-butyldimethylchlorosilane (603 mg) at room temperature for 2 hours. The mixture is evaporated *in vacuo* and the residue is redissolved in ethyl acetate and washed with 1N HCl, water and brine. The organic layer is separated, dried over $MgSO_4$ and evaporated *in vacuo* to give 2.

Step B:

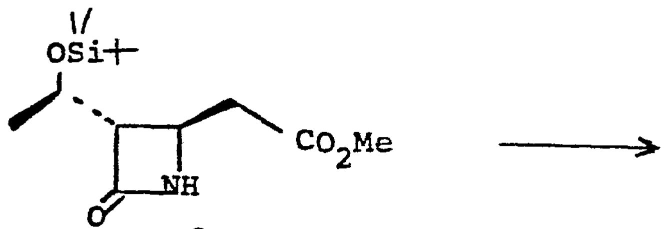

2

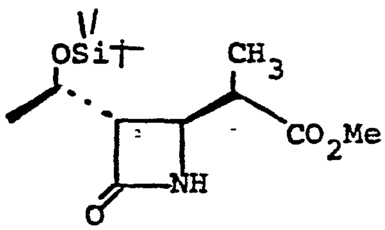

3

+

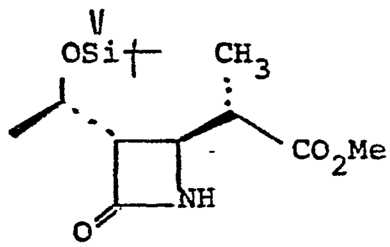

4

Under $N_2$ atmosphere, at -78°C, diisopropylamine (1.68 mg) in 12.5 ml THF is treated with n-BuLi (12.5 ml, 1.6M in hexane) for 10 minutes. To the solution is added THF solution of 2 (1.51 g in 5 ml THF) and the mixture is stirred for 20 minutes, then treated with iodomethane (1.87 ml). After stirring 40 minutes at -78°C, the mixture is allowed to warm to 0°C then hydrolyzed with 0.1N HCl. The mixture is extracted with ethyl acetate. The organic layer is washed with water and brine then dried over $MgSO_4$ and evaporated *in vacuo* to give 2.1 g of isomeric mixture of 3 which are separated by HPLC to give pure ß-methyl isomer 3 and α-methyl isomer 4.

ß-methyl isomer 3: 300 MHz NMR ($CDCl_3$): 0.07 (s), 0.89 (s), 1.18 (d), 1.24 (d), 2.72 (quintet), 3.00 (q), 3.91 (q), 4.22 (m), and 5.83 (s).

α-methyl isomer 4: 200 MHz NMR ($CDCl_3$): 0.08 (s), 0.09 (s), 0.88 (s), 1.26 (d), 2.55 (m), 2.78 (q), 3.70 (q, J=2.1 and 6.5 Hz), 3.74 (s), 4.20 (m), and 6.00 (s).

Step C:

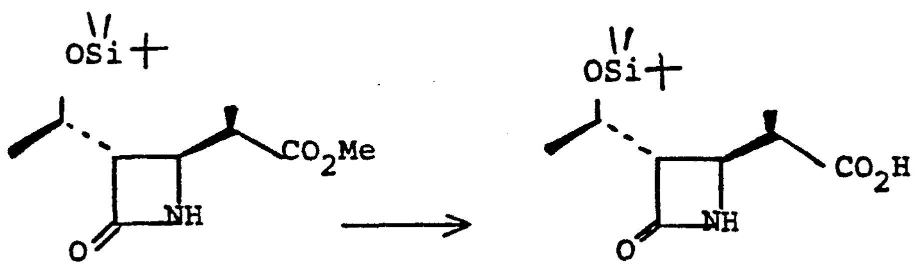

3

5

The methyl ester 3 (1.0 g) is treated with NaOH solution (2.5N, 1.4 ml) in methanol (5 ml) at room temperature for 5 hours. The mixture is acidified with 1N HCl to pH 1.0 to precipitate the desired product 5. The crystalline 5 (0.7 g) collected by filtration. 200 MHz NMR ($CDCl_3$): 0.07 (s), 0.86 (s), 1.20 (d), 1.27 (d), 2.76 (m), 3.04 (q, J=2.1 and 4.2 Hz), 3.96 (q, J=2.1 and 5.0 Hz), 4.22 (m), and 7.37 (s).

Step D:

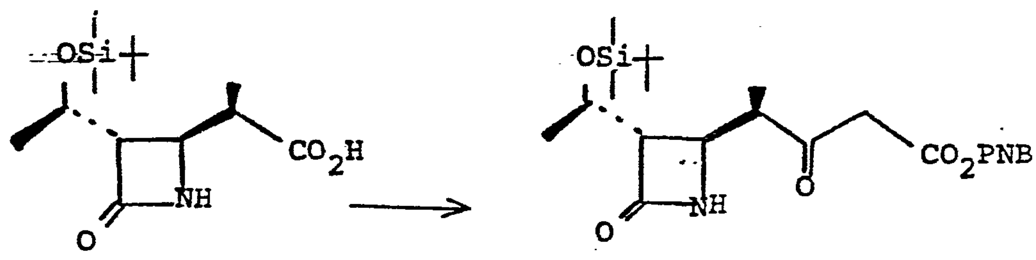

5 6

The azetidinone carboxylic acid 5 (7.5 g) dissolved in 300 ml acetonitrile is treated with carbonyldiimidazole (4.85 g) and stirred at room temperature for 20 minutes. To the solution is added magnesium p-nitrobenzylmalonate (15.0 g) and the mixture is heated at 60°C for 20 hours. The mixture is evaporated *in vacuo* and the residue is taken up in EtOAc which is then washed with 1N HCl, 10% $K_2CO_3$, water and brine. The organic layer is dried over $Na_2SO_4$ then evaporated to give crude product 6 which is purified by a silica gel column (4.3 x 16 cm) eluting with ethyl acetate to give 14.2 g of 6.

Step E:

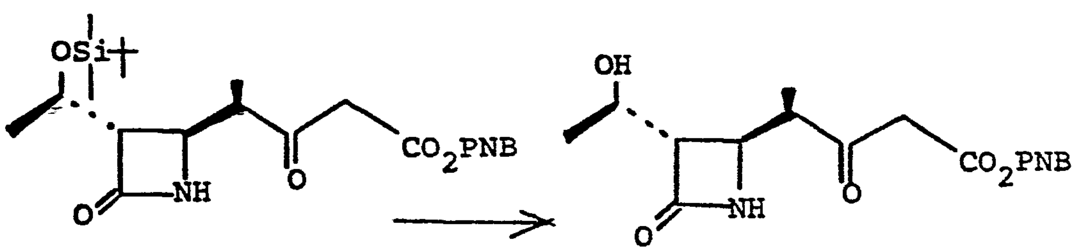

6 7

The silyl azetidinone 6 (14.0 g) is dissolved in 140 ml methanol and treated wtih 6N HCl (14.3 ml) at room temperature for 1 hour. The mixture is evaporated in vacuo and the residue is taken up in ethyl acetate and washed with water. The organic layer is separated, dried over $Na_2SO_4$ and evaporated to give crude product which is purified by crystallization from ether/$CH_2Cl_2$ solution to give 9.8 g of 7. 300 MHz NMR ($CDCl_3$): 1.25 (d), 1.30 (d), 2.90 (q), 2.94 (m), 3.66 (s), 3.68 (q), 3.84 (q), 4.14 (m), 5.28 (s), 6.10 (s), 7.55 (d) and 8.27 (d).

Step F:

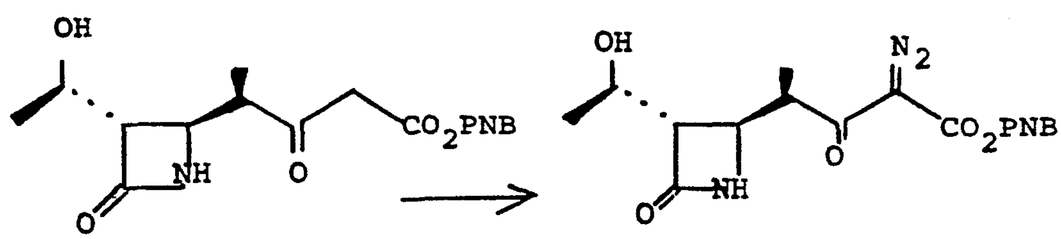

7 8

The ß-keto ester 7 (2.7 g) in acetonitrile (32 ml) is treated with polymeric-$SO_2N_3$ (11.1 g) and triethylamine (3.11 ml) and stirred at room temperature for 3 hours. The mixture is filtered from polymers and the filtrate is chromatographed by a short silica gel column (4.3 x 10 cm) eluting with ethyl acetate to give crystalline 8 (1.81 g) after solvents are removed by evaporation in vacuo. 300 MHz NMR ($CDCl_3$): 1.21 (d), 1.30 (d), 2.64 (d), 2.91 (q), 3.77 (quintet), 3.86 (q), 4.14 (m), 5.36

(s), 6.19 (s), 7.58 (d) and 8.30 (d); ir (film): 2212 $cm^{-1}$ ($N_2$).

Step G:

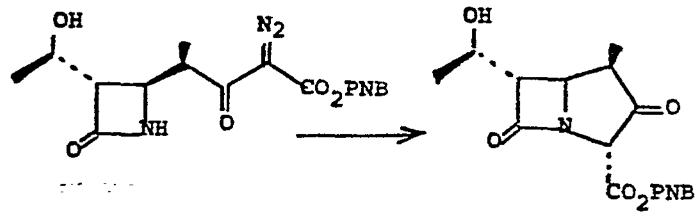

8 9

The diazoazetidinone 8 (100 mg) in 75% ethyl acetate/cyclohexane (2 ml) is heated at reflux. To the solution is added 1 mg of rohdium octanoate. After heating at reflux for 10 minutes, the mixture is evaporated *in vacuo* to give 9 as solid. 200 MHz NMR ($CDCl_3$): 1.23 (d), 1.39 (d), 2.86 (quintet), 3.30 (q, =2.3 and 6.4 Hz), 4.27 (q, J=2.3 and 8.0 Hz), 4.36 (m), 4.78 (s), 5.28 (d), 5.39 (d), 7.59 (d) and 8.28 (d).

Step H:

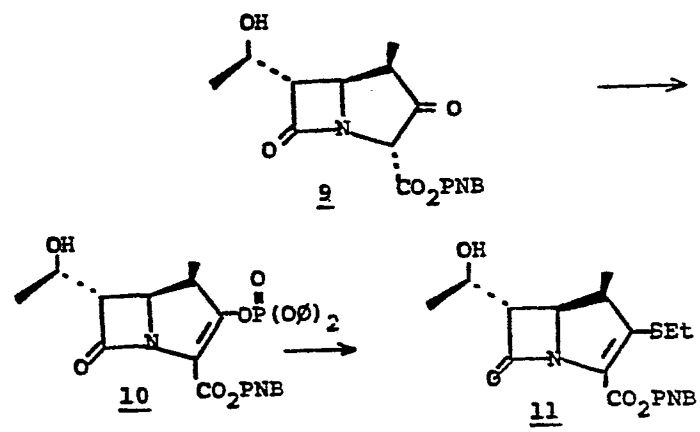

9

10 11

At 0°C, under $N_2$ atmospheres, the bicyclic ß-keto ester 9 (90 mg) is dissolved in acetonitrile (1.0 ml) and treated with diphenyl chlorophosphate (56.6 µl) and diisopropylethylamine (50 µl) for 30 minutes. To the solution is added ethanethiol (48 µl) and diisopropylethylamine (50 µl) and the mixture is kept in the refrigerator for 4 days. Isolation by silica gel plates eluted with ethylacetate give 60.9 mg of product 11. 200 MHz NMR ($CDCl_3$): 1.29 (d), 1.36 (t), 1.40 (d), 2.91 (m), 3.28 (q, J=2.3 and 7.0 Hz), 3.44 (m), 4.04 (q), 4.28 (m), 5.26 (d), 5.56 (d), 7.70 (d) and 8.26 (d).
Mass spect. $M^+$ 406, $(M-44)^+$ 362.

Step I:

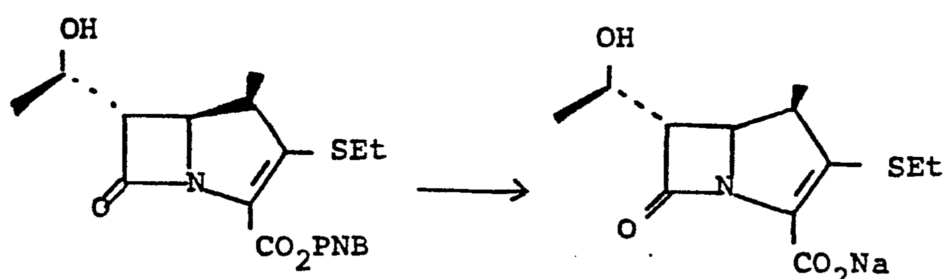

11

12

The carbapenem ester 11 (115 mg), THF (10 ml), EtOH (10 ml), water (7.7 ml) and $NaHCO_3$ (25 mg) are hydrogenated at 50 psi in the presence of 10% Pd/C (57.5 mg) for 3 hours. The mixture diluted with water (56 ml) and filtered from catalyst. The filtrate is concentrated in vacuo to 10 ml then chromatogaphed by a XAD-2 column (2.3 x 15 cm) eluting with D.I. water to give 45 mg of 12 as solids

after concentration and lyophilization. UU $\lambda_{max}^{H_2O}$ 303 nm; 200 MHz NMR ($D_2O$): 1.15 (d), 1.27 (t), 1.32 (d), 2.86 (m), 3.42 (q, J=2.8 and 6.2 Hz), 3.47 (m), 4.20 (J=2.8 and 9.0 Hz), and 4.24 (m).

Step J:

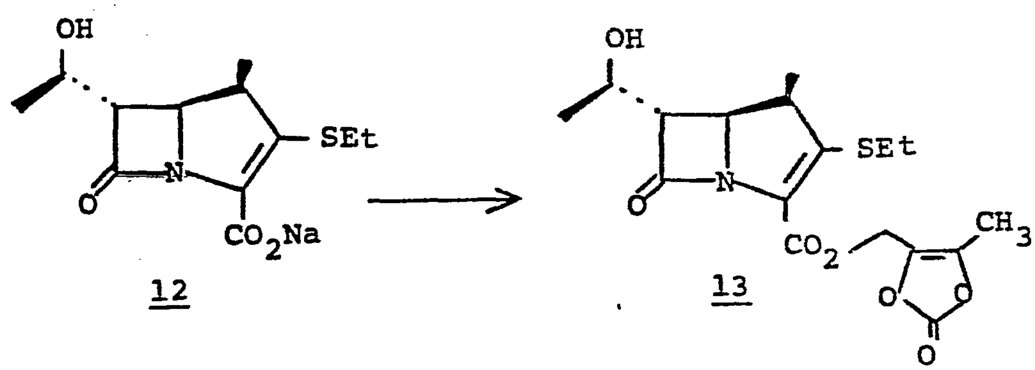

12 13

The sodium salt 12 (45 mg), in DMF (378 µl) is treated with 4-bromomethyl-5-methyl-1,3-dioxolen-2-one (57.6 mg) at room temperature for 2 hours. The mixture is evaporated in vacuo and chromatographed by silica gel plates eluting with ethyl acetate to give 38.1 mg of 13 as solid after removal of solvents. 200 MHz NMR ($CDCl_3$): 1.28 (d), 1.36 (t), 1.38 (d), 2.22 (s), 2.90 (m), 3.15 (q), 3.24 (m), 4.24 (q), 4.25 (m), 4.98 (q) and 5.06 (q).

EXAMPLE 4

Preparation of (5R,6S,8R)-1-ß-methyl-2-cyanomethyl-thio-6-(1-hydroxyethyl)-1-carbapen-2-em-3-carboxylic acid 5-(t-butyl)-2-oxo-1,3-dioxolen-4-yl-methyl ester

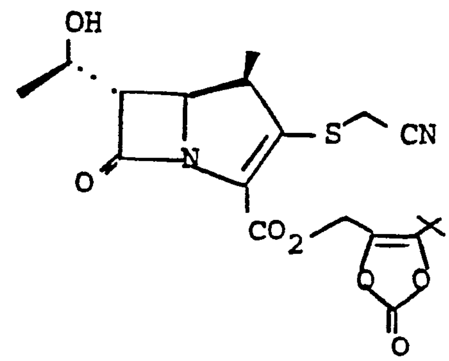

Step A:

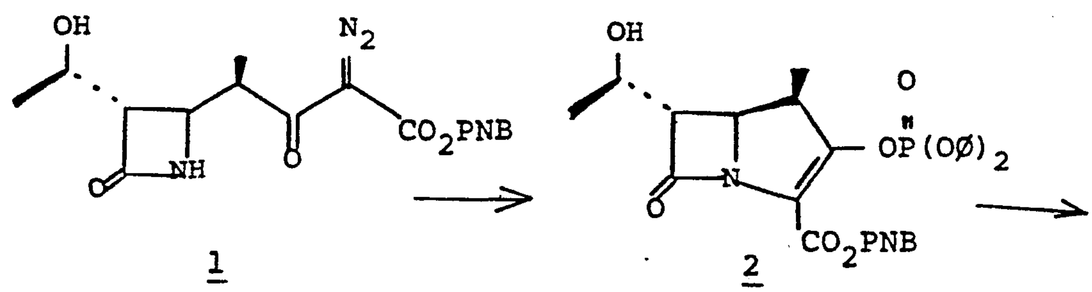

1 2

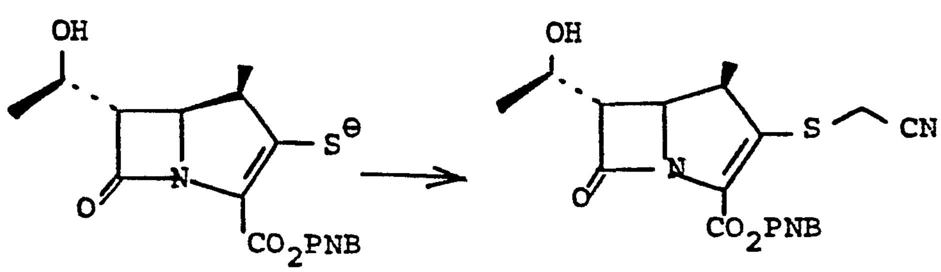

3 4

The diazoazetidinone 1 (100 mg) is heated at reflux in 75% EtOAc/cyclohexane. To the solution is added 0.5 mg of rhodium octanoate and reflux for 10 minutes. The mixture is evaporated *in vacuo* and the residue redissolved in acetonitrile (1.0 ml). At 0°C, to the acetonitrile solution is added diphenylchlorophosphate (56.6 µl) and diisopropylethylamine (49.8 µl) for 20 minutes. The mixture containing vinyl phosphate intermediate is evaporated *in vacuo* and the residue is redissolved in DMF (0.78 ml) and chilled to -35°C in an ethylene/glycol $H_2O$/dry ice bath. To the solution is added sodium hydrogen sulfide (17.5 mg) in 0.76 ml DMF and diisopropyl ethylamine (54.3 µl). The mixture is stirred at -35°C for 1 hour, then treated with chloroacetonitrile (33 µl) and stirred for 30 minutes at -20°C. The mixture is charged onto two 1000 µ silica gel plates which is eluted with 75% EtOAc/cyclohexane to give product 4 (70.5 mg). 200 MHz NMR ($CDCl_3$): 1.34 (d), 1.39 (t), 3.36 (q, J=3.0 and 6.5 Hz), 3.50 (d), 3.76 (d), 3.58 (m), 4.30 (quintet), 4.39 (q), 5.30 (d), 5.53 (d), 7.68 (d) and 8.36 (d).

Step B:

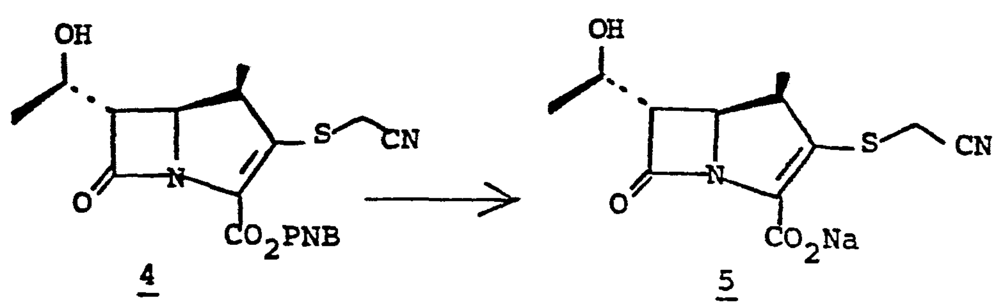

4 5

The ester 4 (20 mg), 10% Pd/C (10 mg), sodium bicarbonate (4.02 mg) in THF (1.71 ml), water (1.34 ml) and ethanol (1.71 ml) are hydrogenated at 50 psi for 1 hour. The mixture is filtered from catalyst and the filtrate is concentrated and purified by a XAD-2 column to give product 2 (9.0 mg) which is lyophilized to give solids.

Step C:

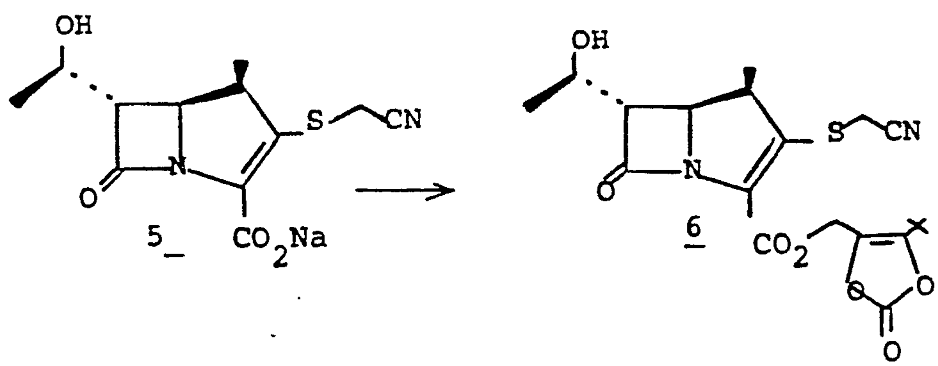

5 6

The sodium salt 5 (4.5 mg) in DMF (40 µl) is treated with 4-bromomethyl-5-(t-butyl)-1,3-dioxolen-2-one (5.8 mg) at room temperature for 2 hours. The mixture is evaporated *in vacuo* then purified by silica gel plates to give ester 6.

EXAMPLE 4A

Preparation of (5R,6S,8R)-1-ß-methyl-2-cyanomethylthio-6-(1-hydroxyethyl)-1-carbapen-2-em-3-carboxylic acid 5-indanyl ester

The sodium salt from Step B, Example 4 (100 mg) is suspended in toluene (10 ml). The mixture is cooled to 0° under $N_2$ and treated with 20 µl of pyridine and 100 mg of oxalyl chloride. The reaction

mixture is stirred for 3 hours and the solvent and excess oxalyl chloride is removed under vacuum. The residue of the crude acid chloride is dissolved in $CH_2Cl_2$ (10 ml) cooled to 0° under $N_2$ and treated with 5-indanol (50 mg) and pyridine 40 µl. The reaction mixture is stirred at 0° for 3 hours and diluted with $CH_2Cl_2$, washed with pH 3 phosphate buffer followed by pH 7 phosphate buffer then dried over $MgSO_4$ and evaporated. The residue is purified by preparative tlc to give the desired product.

EXAMPLE 4B

Preparation of (5R,6S,8R)-1-ß-methyl-2-cyanomethylthio-6-(1-hydroxyethyl)-1-carbapen-2-em-3-carboxylic acid phenyl ester

Following the procedure of Example 4B and substituting phenol for 5-indanol, the above product is obtained.

EXAMPLE 5

Following the procedures described in the foregoing examples and text, the following 1-ß-methylcarbapenem ester I of the present invention are obtained. Examples are summarized in Table I.

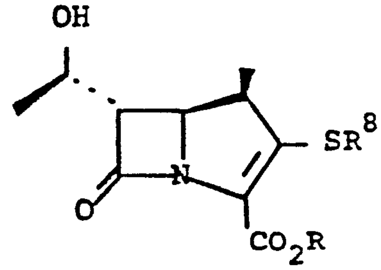

TABLE I

| | R | $R^8$ |
|---|---|---|
| 1 | $-CH_2O\overset{O}{\overset{\|}{C}}C(CH_3)_3$ | 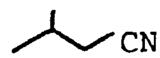 |
| 2 | $-\overset{CH_3}{\overset{\|}{C}}HO\overset{O}{\overset{\|}{C}}C(CH_3)_3$ | 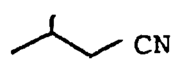 |
| 3 | $-\overset{CH_3}{\overset{\|}{C}}HO\overset{O}{\overset{\|}{C}}OEt$ | 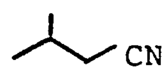 |
| 4 | 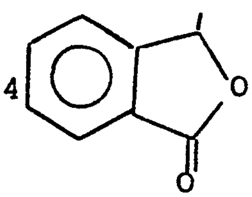 | 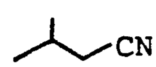 |
| 5 | 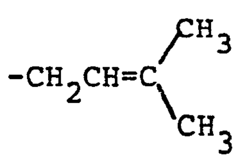 | 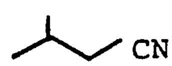 |
| 5a | $-CH_2O\overset{O}{\overset{\|}{C}}OEt$ | 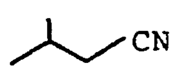 |

| | R | $R^8$ |
|---|---|---|
| 6 | 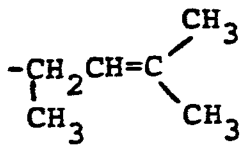 | 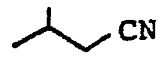 |
| 7 | 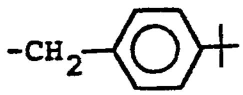 | 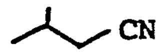 |
| 7a | 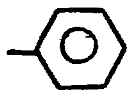 | 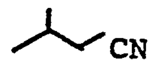 |
| 7b | 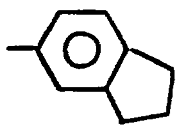 | 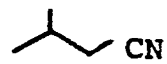 |
| 8 | $CH_2-S-CH_3$ | 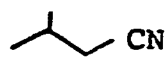 |
| 9 | 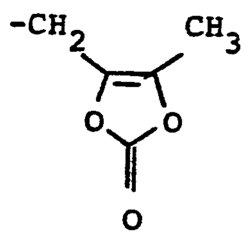 | 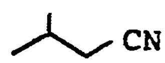 |
| 10 | 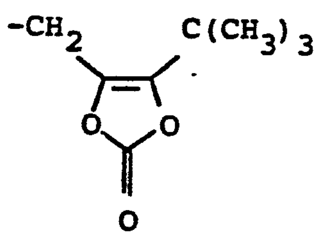 | 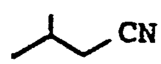 |
| 11 | 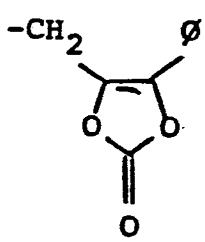 | 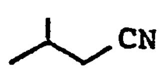 |

| | R | $R^8$ |
|---|---|---|
| 12 | 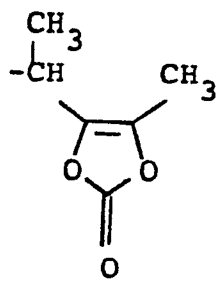 | 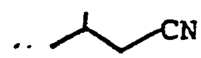 |
| 13 | 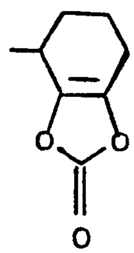 | 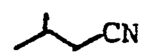 |
| 14 | 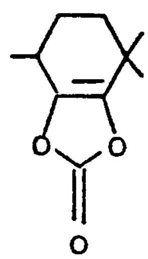 | 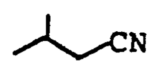 |
| 15 | 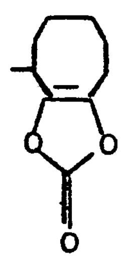 | 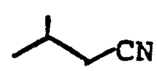 |
| 15a | $CH_2OCH_3$ | 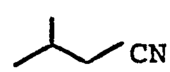 |

| | R | $R^8$ |
|---|---|---|
| 16 | $-CH_2O\overset{O}{\overset{\|}{C}}C(CH_3)_3$ | Et |
| 17 | 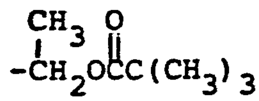 | Et |
| 18 | 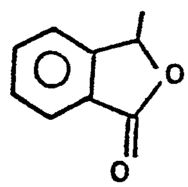 | Et |
| 19 | 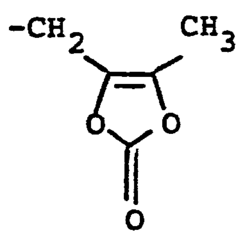 | Et |
| 20 | 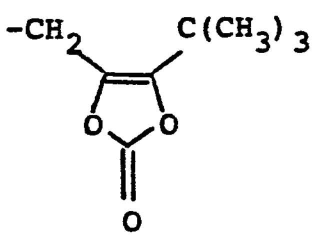 | Et |
| 21 | 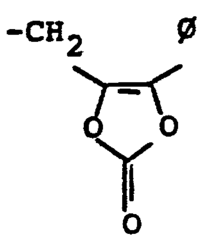 | Et |
| 21a | $CH_2O\overset{O}{\overset{\|}{C}}OEt$ | Et |

| | R | $R^8$ |
|---|---|---|
| 22 | 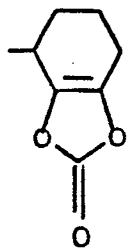 | Et |
| 23 | 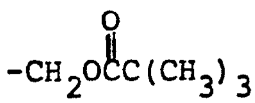 | 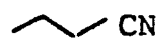 |
| 24 | 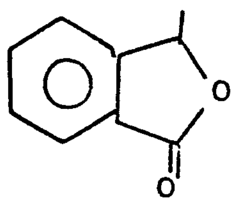 | 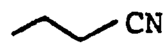 |
| 25 | 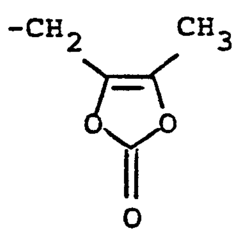 | 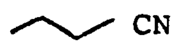 |
| 26 | 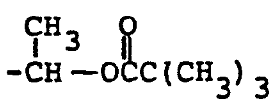 | 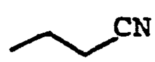 |
| 27 | 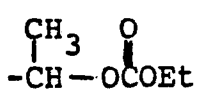 | 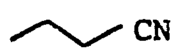 |

| | R | $R^8$ |
|---|---|---|
| 28 | 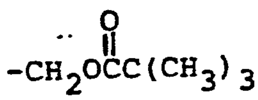 | 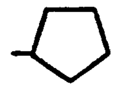 |
| 29 | 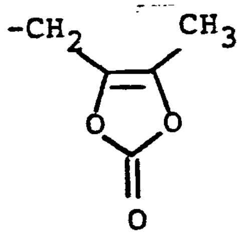 | 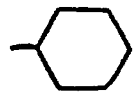 |
| 30 | 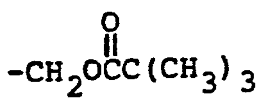 | 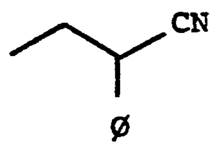 |
| 31 | 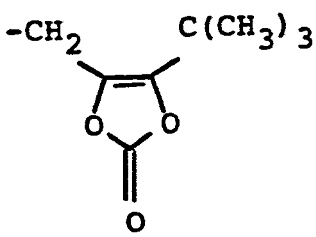 | 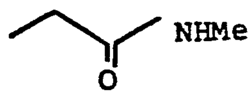 |
| 32 | 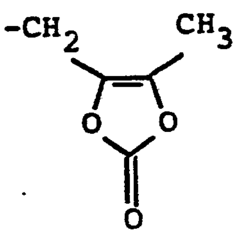 | 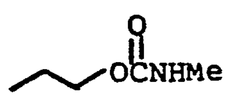 |
| 33 | 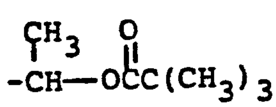 | $CF_3$ |

| | $R$ | $R^8$ |
|---|---|---|
| 34 | 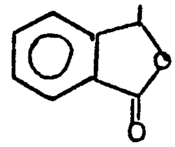 | 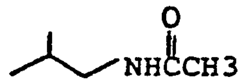 |
| 35 | 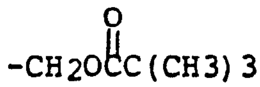 | 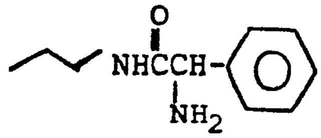 |
| 36 | 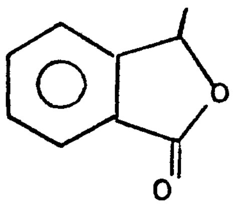 | 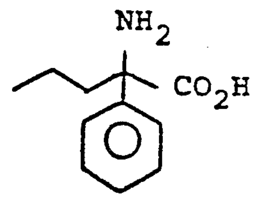 |
| 37 | 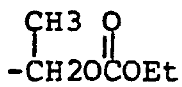 | 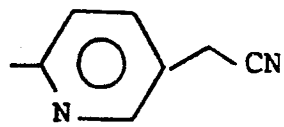 |
| 38 | 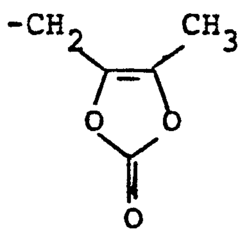 | 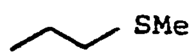 |
| 39 | 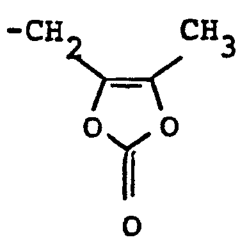 | 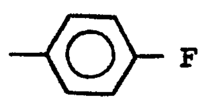 |

| | R | $R^8$ |
|---|---|---|
| 40 | 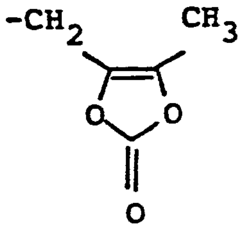 | 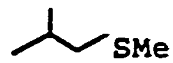 |
| 41 | 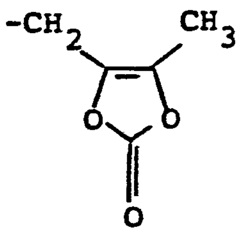 | 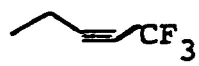 |
| 42 | 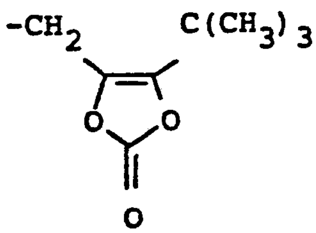 | 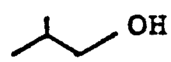 |
| 43 | 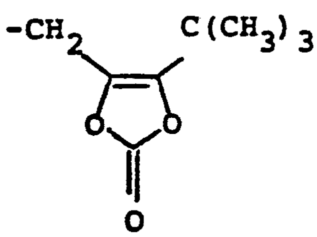 | 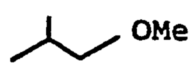 |
| 44 | $CH_2O\overset{O}{\overset{\|}{C}}C(CH_3)_3$ | Et |
| 45 | $CH_2O\overset{O}{\overset{\|}{C}}C(CH_3)_3$ | Me |

| | R | $R^8$ |
|---|---|---|
| 46 | $CH_2O\overset{O}{\overset{\|}{C}}C(CH_3)_3$ | 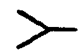 |
| 47 | $CH_2O\overset{O}{\overset{\|}{C}}C(CH_3)_3$ | 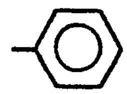 |
| 48 | $CH_2O\overset{O}{\overset{\|}{C}}C(CH_3)_3$ | 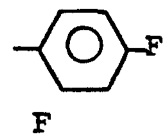 |
| 49 | $CH_2O\overset{O}{\overset{\|}{C}}C(CH_3)_3$ | 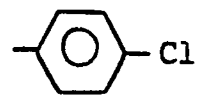 |
| 50 | $CH_2O\overset{O}{\overset{\|}{C}}C(CH_3)_3$ | 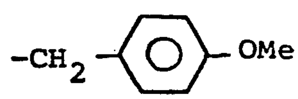 |
| 51 | $CH_2O\overset{O}{\overset{\|}{C}}C(CH_3)_3$ | 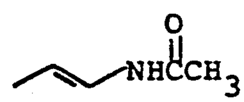 |
| 52 | $CH_2O\overset{O}{\overset{\|}{C}}C(CH_3)_3$ | 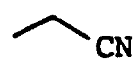 |
| 53 | $CH_2O\overset{O}{\overset{\|}{C}}C(CH_3)_3$ | 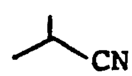 |

| | R | $R^8$ |
|---|---|---|
| 54 | $CH_2O\overset{O}{\overset{\parallel}{C}}C(CH_3)_3$ | 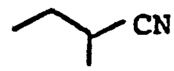 |
| 55 | $CH_2O\overset{O}{\overset{\parallel}{C}}C(CH_3)_3$ | 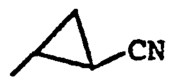 |
| 56 | $CH_2O\overset{O}{\overset{\parallel}{C}}C(CH_3)_3$ | 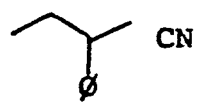 |
| 57 | $CH_2O\overset{O}{\overset{\parallel}{C}}C(CH_3)_3$ | 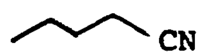 |
| 58 | $CH_2O\overset{O}{\overset{\parallel}{C}}C(CH_3)_3$ | 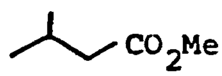 |
| 59 | $CH_2O\overset{O}{\overset{\parallel}{C}}C(CH_3)_3$ | 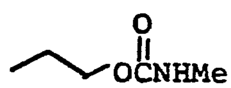 |
| 60 | $CH_2O\overset{O}{\overset{\parallel}{C}}C(CH_3)_3$ | 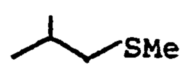 |
| 61 | $CH_2O\overset{O}{\overset{\parallel}{C}}C(CH_3)_3$ | 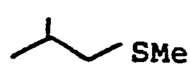 |
| 62 | $CH_2O\overset{O}{\overset{\parallel}{C}}C(CH_3)_3$ | 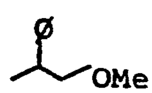 |

| | R | $R^8$ |
|---|---|---|
| 63 | $CH_2O\overset{O}{\overset{\|}{C}}C(CH_3)_3$ | 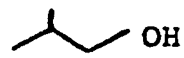 |
| 64 | $CH_2O\overset{O}{\overset{\|}{C}}C(CH_3)_3$ | $CF_3$ |
| 65 | $CH_2O\overset{O}{\overset{\|}{C}}C(CH_3)_3$ | 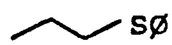 |
| 66 | $CH_2O\overset{O}{\overset{\|}{C}}C(CH_3)_3$ | 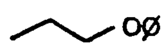 |
| 67 | $CH_2O\overset{O}{\overset{\|}{C}}C(CH_3)_3$ | 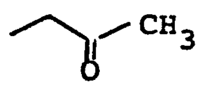 |
| 68 | $CH_2O\overset{O}{\overset{\|}{C}}C(CH_3)_3$ | 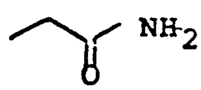 |
| 69 | $CH_2O\overset{O}{\overset{\|}{C}}C(CH_3)_3$ | 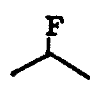 |
| 70 | $CH_2O\overset{O}{\overset{\|}{C}}C(CH_3)_3$ | 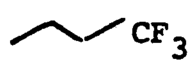 |
| 71 | $CH_2O\overset{O}{\overset{\|}{C}}C(CH_3)_3$ | 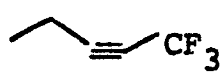 |

| | R | $R^8$ |
|---|---|---|
| 72 | $CH_2O\overset{O}{\overset{\|}{C}}C(CH_3)_3$ | 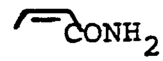 |
| 73 | $CH_2O\overset{O}{\overset{\|}{C}}C(CH_3)_3$ | 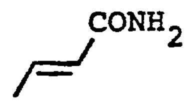 |
| 74 | $CH_2O\overset{O}{\overset{\|}{C}}C(CH_3)_3$ | 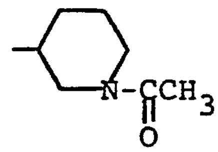 |
| 75 | $CH_2O\overset{O}{\overset{\|}{C}}C(CH_3)_3$ | 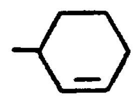 |
| 76 | $CH_2O\overset{O}{\overset{\|}{C}}C(CH_3)_3$ | 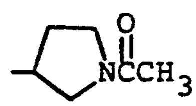 |
| 77 | $CH_2O\overset{O}{\overset{\|}{C}}C(CH_3)_3$ | 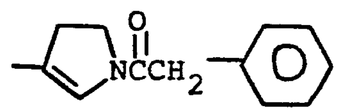 |
| 78 | $CH_2OCC(CH_3)_3$ | 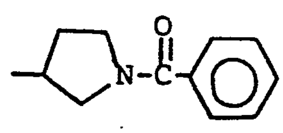 |
| 79 | $CH_2O\overset{O}{\overset{\|}{C}}C(CH_3)_3$ | 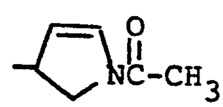 |

| | | |
|---|---|---|
| 80 | $CH_2O\overset{O}{\overset{\|}{C}}C(CH_3)_3$ | 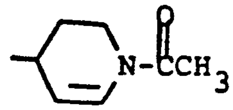 |
| 81 | $CH_2O\overset{O}{\overset{\|}{C}}C(CH_3)_3$ | 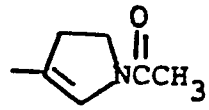 |
| 82 | $CH_2O\overset{O}{\overset{\|}{C}}C(CH_3)_3$ | 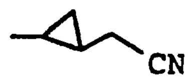 |
| 83 | 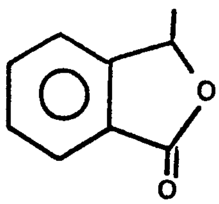 | $R^8$ of compound 1 to 82 |
| 84 | $-\overset{CH_3}{\overset{\|}{C}}H-O-\overset{O}{\overset{\|}{C}}-OEt$ | 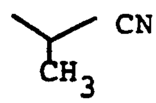 |
| 85 | $-\overset{CH_3}{\overset{\|}{C}}H-O\overset{O}{\overset{\|}{C}}C(CH_3)_3$ | 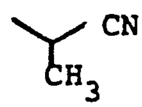 |
| 86 | $-\overset{CH_3}{\overset{\|}{C}}H-O-\overset{O}{\overset{\|}{C}}-C(CH_3)_3$ | 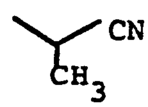 |
| 87 | 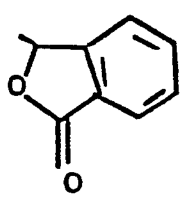 | 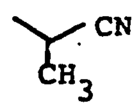 |

0113101



| | | |
|---|---|---|
| 88 | 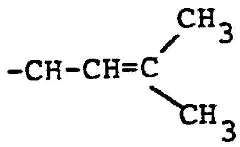 | 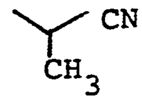 |
| 89 | 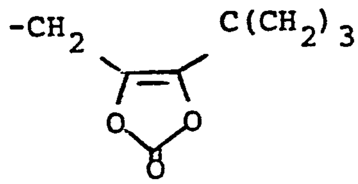 | 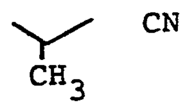 |
| 90 | 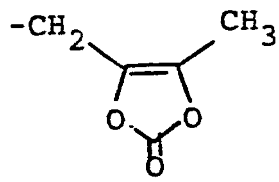 | 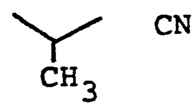 |
| 91 | 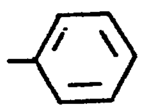 | 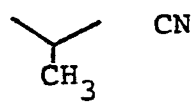 |
| 92 | 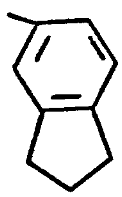 | 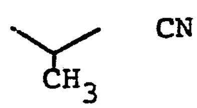 |
| 93 | 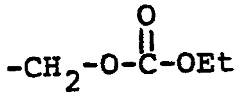 | 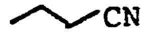 |
| 94 | 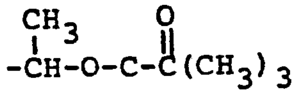 | 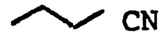 |

95 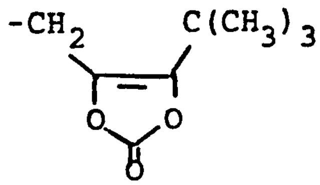 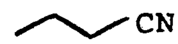

96. 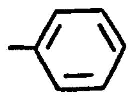 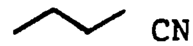

97 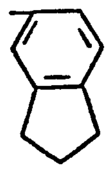 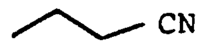

98 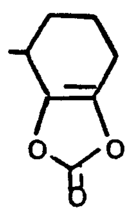 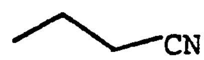

99 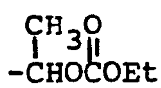 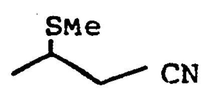

100 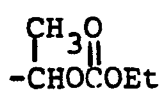 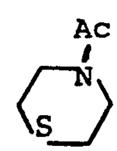

EXAMPLE 6

Preparation of Pharmaceutical Compositions

One such unit dosage form is prepared by mixing 120 mg of the compound of Example 5, Compound 4, with 20 mg of lactose and 5 mg of magnesium stearate and placing the 145 mg mixture into a No. 3 gelatin capsule. Similarly, by employing more of the active ingredient and less lactose, other dosage forms can be put up in No. 3 gelatin capsules, and, should it be necessary to make more than 145 mg of ingredients together, larger capsules such as compressed tablets and pills can be prepared. The following examples are illustrative of the preparation of pharmaceutical formulations:

| TABLET | PER TABLET |
|---|---|
| Compound 4 | 125 mg. |
| Cornstarch, U.S.P. | 6 mg. |
| Dicalcium Phosphate | 192 mg. |
| Lactose, U.S.P. | 190 mg. |
| Magnesium Stearate | Balance/800 mg. |

The active ingredient is blended with the dicalcium phosphate, lactose and about half of the cornstarch. The mixture is then granulated with 15% cornstarch paste (16 mg) and rough-screened. It is dried at 45°C and screened again through No. 16 screens. The balance of the cornstarch and magnesium stearate is added and the mixture is compressed into tablets, approximately 0.5 inch in diameter each weighing 800 mg.

WHAT IS CLAIMED IS:

1. A compound having the structure:

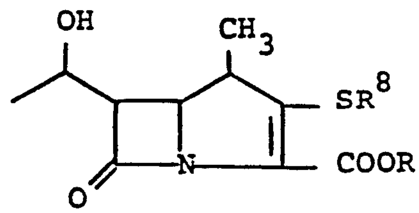

wherein R is selected from the group consisting of:

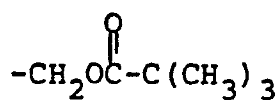

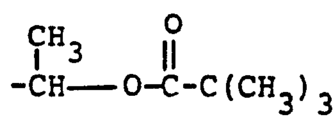

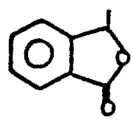

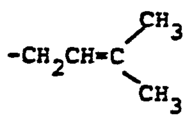

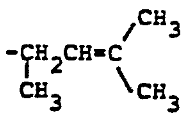

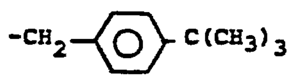

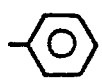

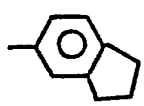

$-CH_2-S-CH_3$

$-CH_2-O-CH_3$

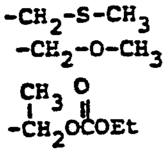

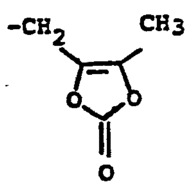

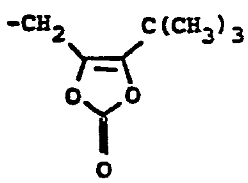

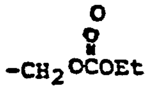

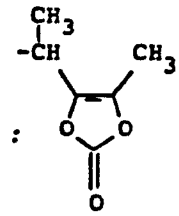

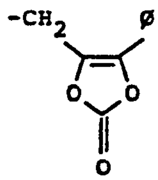

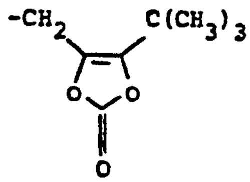

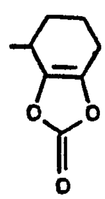

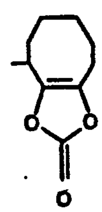

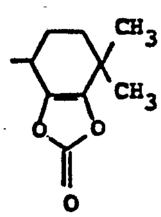

$R^8$ is substituted or unsubstituted: alkyl, alkenyl, alkynyl, or cyclic alkyl, alkenyl, or alkynyl, having 1-6 carbon atoms, phenyl, pyridyl; wherein the substituent or substituents are selected from: phenyl, pyridyl, cyano, fluoro, chloro, hydroxy, methylthio, phenylthio, methoxy, phenoxy, methoxycarbonyl, acetoxyl, N-methylcarbamoyl, N-methylcarbamoyloxy and N-acylamino.

2. A compound according to Claim 1 wherein $R^8$ is selected from the group consisting of:

$CH_3$

$CH_2CH_3$

$CH(CH_3)_2$

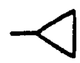

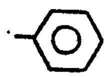

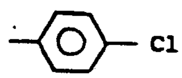

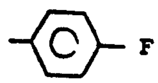

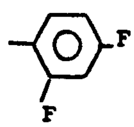

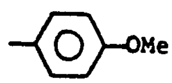

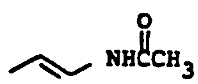

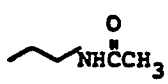

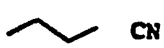

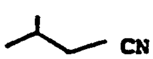

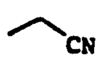

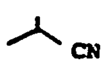

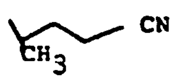

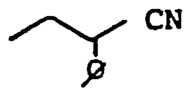

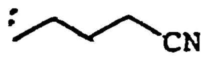

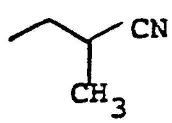

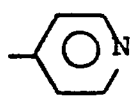

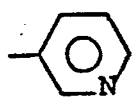

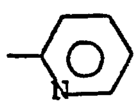

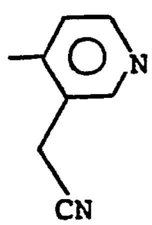

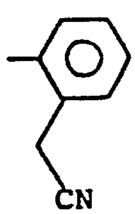

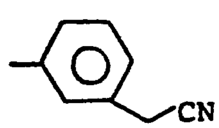

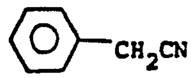

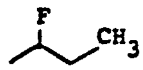

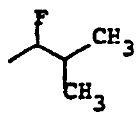

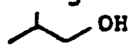

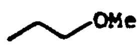

Me = $CH_3$

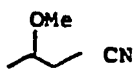

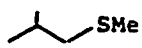

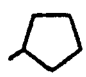

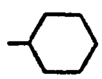

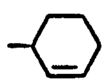

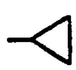

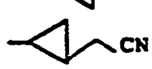

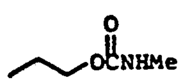

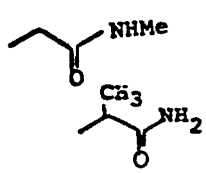

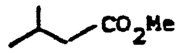

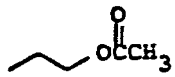

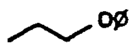

Ø = phenyl

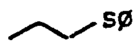

$-CF_3$

$-CH_2C\equiv CCH_3$

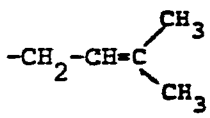

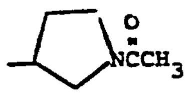

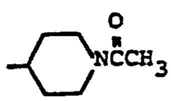

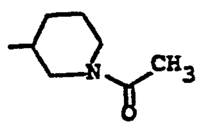

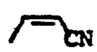

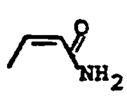

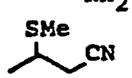

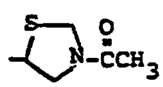

3. A process for preparing a compound having the structure:

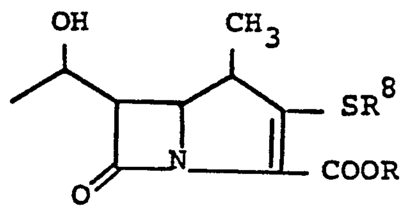

wherein R is selected from the group consisting of:

$-CH_2O\overset{O}{\overset{\|}{C}} - C(CH_3)_3$

$-\overset{CH_3}{\overset{|}{C}}H-O-\overset{O}{\overset{\|}{C}} - C(CH_3)_3$

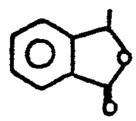

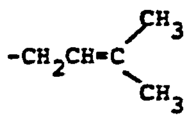

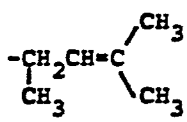

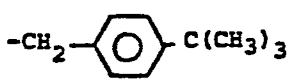

$-CH_2-S-CH_3$

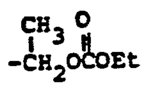

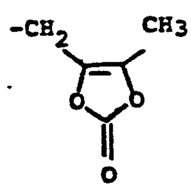

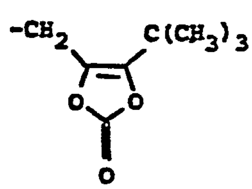

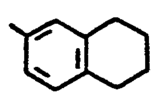

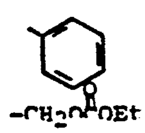

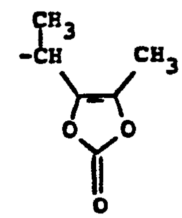

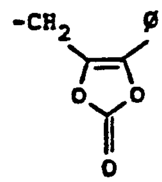

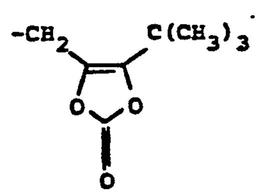

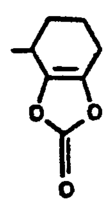

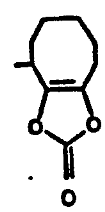

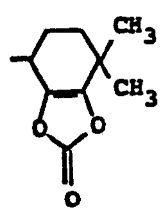

$R^8$ is substituted or unsubstituted: alkyl, alkenyl, alkynyl, or cyclic alkyl, alkenyl, or alkynyl, having 1-6 carbon atoms, phenyl, pyridyl; wherein the substituent or substituents are selected from: phenyl, pyridyl, cyano, fluoro, chloro, hydroxy, methylthio, phenylthio, methoxy, phenoxy, methoxycarbonyl, acetoxyl, N-methylcarbamoyl, N-methylcarbamoyloxy and N-acylamino comprising treating:

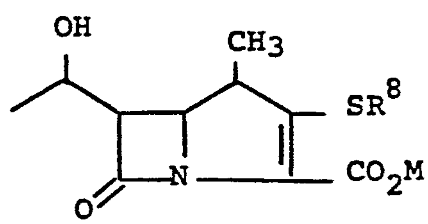

with $RX^1$ wherein $X^1$ is a leaving group and M is an alkali metal cation.

4. An antibacterial pharmaceutical composition comprising a therapeutically effective amount of a compound according to Claim 1 and a pharmaceutical carrier therefor.

5. A compound according to Claim 1 or 2 having the 5<u>R</u>,6<u>S</u>,8<u>R</u>, 1-β-methyl configuration.

European Patent Office

# EUROPEAN SEARCH REPORT

0113101
Application number

EP 83112827.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP - A1 - 0 010 317 (MERCK)<br>* Pages 1-3; claims 1-5,8,25 *<br>-- | 1-4 | C 07 D 487/04<br>A 61 K 31/40 |
| Y | EP - A1 - 0 060 077 (BEECHAM)<br>* Page 18; claims 1,17 *<br>-- | 1,4 | |
| P,Y | EP - A2 - 0 089 139 (BEECHAM)<br>* Page 10; claims 1,5,6 *<br>---- | 1-4 | |
| | | | TECHNICAL FIELDS SEARCHED (Int. Cl. 3) |
| | | | C 07 D 487/00 |

The present search report has been drawn up for all claims

| Place of search | Date of completion of the search | Examiner |
|---|---|---|
| VIENNA | 07-03-1984 | JANISCH |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82